# EUROPEAN PATENT APPLICATION

(11) **EP 4 556 483 A1**
(43) Date of publication of application: **21.05.2025**
(21) Application number: 24846045.3
(22) Date of filing: 24.07.2024
(51) Int. Cl.: C07K 14/34, C12N 15/01, C12N 15/77, C12P 13/08

(54) **PROTEIN VARIANT AND METHOD FOR PRODUCING L-LYSINE USING SAME**

(30) Priority: 24.07.2023 KR 20230096086
(71) Applicant: CJ CheilJedang Corporation, Seoul 04560 (KR)
(72) Inventor: YOON, Jeong-Hye, Seoul 04560 (KR); KIM, Gyuree, Seoul 04560 (KR); KIM, Hye Mi, Seoul 04560 (KR); SHIN, Kwang Soo, Seoul 04560 (KR); SEO, Chang Il, Seoul 04560 (KR); JUNG, Moo Young, Seoul 04637 (KR)
(74) Representative: Meissner Bolte Partnerschaft mbB
(86) International application number: PCT/KR2024/010750
(87) International publication number: WO 2025/023743

(57) **Abstract**

Provided are a protein variant, a microorganism of the genus of *Corynebacterium* comprising the protein variant and a method of producing L-lysine using the microorganism in the present disclosure.

## Description

### [TECHNICAL FIELD]

### Cross-reference to related application(s)

The present disclosure claims the benefit of the priority based on Korean Patent Application No. 10-2023-0096086 filed on July 24, 2023, and the entire contents disclosed in the document of the corresponding Korean patent application are incorporated as a part of the present disclosure.

Throughout the present disclosure, numerous papers and patent documents are referenced and citations thereof are indicated. The disclosures of the cited papers and patent documents are incorporated into the present disclosure in their entirety by reference to more clearly describe the level of the technical field to which the present invention pertains and the contents of the present invention.

The present disclosure relates to a protein variant, a microorganism of the genus of *Corynebacterium* comprising the protein variant, and a method of producing L-lysine using the microorganism.

### [BACKGROUND ART]

In order to produce L-amino acids and other useful substances, various studies are being conducted to develop high-efficiency production microorganisms and fermentation process technologies. For example, target substance-specific approach methods such as increasing expression of genes encoding enzymes involved in L-lysine biosynthesis or removing genes unnecessary for biosynthesis are being mainly used (WO2008-082181 A1).

However, according to an increase in demand of L-lysine, research to effectively increase the productivity of L-lysine is still needed.

### [DISCLOSURE]

### [TECHNICAL PROBLEM]

One object of the present disclosure is to provide a protein variant.

Another object of the present disclosure is to provide a polypeptide encoding the protein variant.

Other object of the present disclosure is to provide a microorganism of the genus of *Corynebacterium,* comprising at least one selected from the group consisting of the protein variant and the polynucleotides encoding the same.

Other object of the present disclosure is to provide a composition for producing L-lysine, comprising the microorganism of the genus of *Corynebacterium.*

Other object of the present disclosure is to provide a method of producing L-lysine, comprising culturing the microorganism of the genus of *Corynebacterium* in a medium.

Other object of the present disclosure is to provide a use for producing L-lysine of the microorganism.

### [TECHNICAL SOLUTION]

Description thereof in detail is as follows. On the other hand, each description, aspect and embodiment disclosed in the present disclosure can also be applied to each other description, aspect and embodiment. In other words, all combinations of various elements disclosed in the present disclosure belong to the scope of the present disclosure. In addition, the scope of the present disclosure is not to be considered limited by the specific description described below. Furthermore, those skilled in the art can recognize or confirm numerous equivalents to the specific aspects of the present application disclosed in the present application using only common experiments. In addition, these equivalents are intended to be included in the present application.

One aspect of the present disclosure provides a protein variant comprising an amino acid sequence in which the amino acid corresponding to the 75th position from the N-terminus in the amino acid sequence of SEQ ID NO: 105 is substituted with an amino acid different from the original amino acid, or an amino acid sequence in which the amino acid corresponding to the 35th position from the N-terminus in the amino acid sequence of SEQ ID NO: 106 is substituted with an amino acid different from the original amino acid.

In the present disclosure, the sequence of SEQ ID NO: 105 is as follows:

MVSQTERQHAIASLLAPTGAVSVGDLAEHFHVTTETVRRDLRIMESLGLLQRVHGGAIS

PEPMGTSPPRLKPALGKGMPPEPRVLELAETAVSLITPLARSIFLDSGLACTAIATVLGDPPEDAR

WTVVTSSPGAVIALSATDATSTVVLHGQVHGNCSSIIGSTAVDMISQLRADIAFVEVDAIQSDTS

LCTFFPETIPIKQAMIKNAAFTVAVLSPRSPQDQELQLLKHPFSTLADFDALVTDDHTLDFPVLPD

HNFQVVTP.

In the present disclosure, the sequence of SEQ ID NO: 106 is as follows:

MTTNAPDGATNNINNAHSGAVGKPKVQLSDAEIQEYTAAFAGTTTTKPWELEVTTKFL
KKIAWVAVVVIMAVHIFMGAVVDVDFTGAAVTFVDTLAFPALGIIFSVLVFLGLTRPRVRANEDG
VEVRNFIGTRFYPWVVIYGMSFPKGSSVARLELPDFEFVPMWAFQSRDGEDVVRAVATFRDLE
NKYMPED.

In one embodiment, the afore-mentioned protein variant of the present disclosure may be essentially consisting of an amino acid sequence in which the amino acid corresponding to the 75th position from the N-terminus in the amino acid sequence of SEQ ID NO: 105 is substituted with an amino acid different from the original amino acid, or an amino acid sequence in which the amino acid corresponding to the 35th position from the N-terminus in the amino acid sequence of SEQ ID NO: 106 is substituted with an amino acid different from the original amino acid. In another embodiment, the afore-mentioned protein variant of the present disclosure may be consisting of an amino acid sequence in which the amino acid corresponding to the 75th position from the N-terminus in the amino acid sequence of SEQ ID NO: 105 is substituted with an amino acid different from the original amino acid, or an amino acid sequence in which the amino acid corresponding to the 35th position from the N-terminus in the amino acid sequence of SEQ ID NO: 106 is substituted with an amino acid different from the original amino acid.

In the present disclosure, the amino acid sequence of SEQ ID NO: 105 may be the amino acid sequence of FruR (transcriptional regulator of sugar metabolism) protein. In one embodiment, the protein may be a protein encoded by NCg11859 gene.

In the present disclosure, the amino acid sequence of SEQ ID NO: 106 may be an amino acid sequence of a protein encoded by NCg11531 gene.

In one embodiment, the amino acid corresponding to the 75th position from the N-terminus in the amino acid sequence of SEQ ID NO: 105 may be glycine (Gly, G), but not limited thereto.

In one embodiment, the amino acid corresponding to the 35th position from the N-terminus in the amino acid sequence of SEQ ID NO: 106 may be glutamic acid (Glu, E), but not limited thereto.

In one embodiment, the amino acid corresponding to the 75th position from the N-terminus in the amino acid sequence of SEQ ID NO: 105 may be substituted with valine (Val, V), but not limited thereto.

In one embodiment, the amino acid corresponding to the 35th position from the N-terminus in the amino acid sequence of SEQ ID NO: 106 may be substituted with lysine (Lys, K), but not limited thereto.

The protein variant of the present disclosure may have activity to increase L-lysine productivity compared to a wild-type protein (polypeptide).

The protein variant of the present disclosure may comprise an amino acid sequence in which the amino acid corresponding to the 75th position from the N-terminus in the amino acid sequence of SEQ ID NO: 105 is substituted with an amino acid (for example, valine) different from the original amino acid, or an amino acid sequence in which the amino acid corresponding to the 35th position from the N-terminus in the amino acid sequence of SEQ ID NO: 106 is substituted with an amino acid (for example, lysine) different from the original amino acid, or comprise an amino acid sequence having homology or identity of at least 70%, 75%, 80%, 85%, 90%, 95%, 96%, 97%, 98%, 99%, 99.5%, or 99.7% or more to said amino acid sequence. In addition, it is obvious that even a variant having an amino acid sequence in which some sequences are deleted, modified, substituted, conservatively substituted, or added is included in the scope of the present disclosure, as long as it is an amino acid sequence which has such homology or identity and shows efficacy (efficacy of increasing L-lysine production) corresponding to the variant of the present disclosure.

For example, they are cases of having addition or deletion of a sequence which does not change the function of the variant of the present disclosure, naturally occurring mutation, silent mutation, or conservative substitution at the N-terminus, C-terminus and/or inside the amino acid sequences.

The "conservative substitution" refers to substituting one amino acid with an amino acid different from the original amino acid having similar structural and/or chemical characteristics. This amino acid substitution may occur generally based on the similarity in polarity, charge, solubility, hydrophobicity, hydrophilicity, and/or amphipathic nature of a residue. Commonly, the conservative substitution may hardly affect or not affect activity of a protein or polypeptide.

In the present disclosure, the term, "variant" refers to a polypeptide which is different from the amino acid sequence before mutation of the variant, as at least one amino acid is under conservative substitution and/or modification, but functions or properties are maintained. Such a variant may be commonly identified by modifying at least one amino acid among the amino acid sequence of the polypeptide, and evaluating the properties of the modified polypeptide. In other words, the ability of the variant may increase, or be not changed, or decrease, compared the polypeptide before mutation. In addition, some variants may include variants in which at least one part such as an N-terminal leader sequence or transmembrane domain is removed. Other variants may include variants in which some parts from the N- and/or C-terminus of a mature protein are removed. The term "variant" may be interchangeably used with terms such as mutant, modification, modified polypeptide, mutated protein, mutation and variant and the like (modification, modified polypeptide, modified protein, mutant, mutein, divergent, etc. as English expressions), and even if it is a term used as the mutated meaning, it is not limited thereto.

In addition, the variant may comprise deletion or addition of amino acids having minimal effects on the properties and secondary structure of the polypeptide. For example, a signal (or leader) sequence related to co-translational or post-translational translocation of proteins may be conjugated at the N-terminus.

Furthermore, the variant may be conjugated with another sequence or a linker to be identified, purified, or synthesized.

In one embodiment, the afore-mentioned protein variant of the present disclosure may have homology or identity of 90%, 91%, 93%, 94%, 95%, 96%, 97%, 98%, or 99% or more, less than 100% to the amino acid sequence of SEQ ID NO: 105, or may have homology or identity of 90%, 91%, 93%, 94%, 95%, 96%, 97%, 98%, or 99% or more, less than 100% to the amino acid sequence of SEQ ID NO: 106, but be not limited thereto. In other words, the afore-mentioned protein variant of the present disclosure may have the amino acid sequence identity of 90%, 91%, 93%, 94%, 95%, 96%, 97%, 98%, or 99% or more, less than 100%, respectively, to the amino acid sequence of SEQ ID NO: 105 or SEQ ID NO: 106, but be not limited thereto.

Another aspect of the present disclosure provides a protein variant, comprising at least one amino acid sequence selected from the group consisting of amino acid sequences of SEQ ID NO: 1, SEQ ID NO: 3 to SEQ ID NO: 5, SEQ ID NO: 7 to SEQ ID NO: 16, and SEQ ID NO: 18. In one embodiment, the protein variant may be a protein variant comprising the amino acid sequence of SEQ ID NO: 13 or SEQ ID NO: 15. In other embodiment, the protein variant may be a protein variant comprising the amino acid sequence of SEQ ID NO: 13 or SEQ ID NO: 15. In other embodiment, the protein variant may be essentially consisting of the amino acid sequence of SEQ ID NO: 13 or SEQ ID NO: 15. In other embodiment, the protein variant may be consisting of the amino acid sequence of SEQ ID NO: 13 or SEQ ID NO: 15.

The protein variant of the present disclosure may comprise any one amino acid sequence selected from the group consisting of amino acid sequences of SEQ ID NO: 1, SEQ ID NO: 3 to SEQ ID NO: 5, SEQ ID NO: 7 to SEQ ID NO: 16, and SEQ ID NO: 18, or comprise an amino acid sequence having homology or identity of at least 70%, 75%, 80%, 85%, 90%, 95%, 96%, 97%, 98%, 99%, 99.5%, 99.7% or 99.9% or more to said amino acid sequence. In addition, it is obvious that even a variant having an amino acid sequence in which some sequences are deleted, modified, substituted, conservatively substituted, or added is included in the scope of the present disclosure, as long as it is an amino acid sequence which has such homology or identity and shows efficacy (efficacy of increasing L-lysine production) corresponding to the variant of the present disclosure.

In the present disclosure, the amino acid sequence of SEQ ID NO: 1 may be a sequence of a protein variant in which the 184th amino acid of the amino acid sequence of ABC transporter permease is substituted from asparagine (Asn, N) to isoleucine (lle, I). The ABC transporter permease may a protein encoded by NCg10030 gene.

In the present disclosure, the amino acid sequence of SEQ ID NO: 3 may be a sequence of a protein variant in which the 86th amino acid of nth (Endolll-related endonuclease) protein is substituted from alanine (Ala, A) to valine (Val, V). The nth protein may be a protein encoded by NCgl0288 gene.

In the present disclosure, the amino acid sequence of SEQ ID NO: 4 may be a sequence of a protein variant in which the 363th amino acid of wzz (chromosome partitioning ATPase) protein is substituted from glutamic acid (Glu, E) to Aspartic acid (Asp, D). The wzz protein may be a protein encoded by NCgl0337 gene.

In the present disclosure, the amino acid sequence of SEQ ID NO: 5 may be a sequence of a protein variant in which the 210th amino acid of NusG (transcription antitermination protein NusG) protein is substituted from asparagine (Asn, N) to aspartic acid (Asp, D). The NusG protein may be a protein encoded by NCgl0458 gene.

In the present disclosure, the amino acid sequence of SEQ ID NO: 7 may be a sequence of a protein variant in which the 23th amino acid of a protein (hypothetical protein) encoded by NCgl0686 gene is substituted from valine (Val, V) to alanine (Ala, A).

In the present disclosure, the amino acid sequence of SEQ ID NO: 8 may be a sequence of a protein variant in which the 1112th amino acid of Type II restriction enzyme, methylase subunits protein is substituted from leucine (Leu, L) to phenylalanine (Phe, F). The Type II restriction enzyme, methylase subunits protein may be a protein encoded by NCgl0706 gene.

In the present disclosure, the amino acid sequence of SEQ ID NO: 9 may be a sequence of a protein variant in which the 101th amino acid of mctC (Na+/proline, Na+/pantothenate symporter or related permease) protein is substituted from tryptophan (Trp, W) to arginine (Arg, R). The mctC protein may be a protein encoded by NCg10799 gene.

In the present disclosure, the amino acid sequence of SEQ ID NO: 10 may be a sequence of a protein variant in which the 461th amino acid of atpD (ATP synthase F0F1 subunit beta) protein is substituted from aspartic acid (Asp, D) to glutamic acid (Glu, E). The atpD protein may be a protein encoded by NCg11165 gene.

In the present disclosure, the amino acid sequence of SEQ ID NO: 11 may be a sequence of a protein variant in which the 456th amino acid of leuC (isopropylmalate isomerase large subunit) protein is substituted from glycine (Gly, G) to aspartic acid (Asp, D). The leuC protein may be a protein encoded by NCgl1262 gene.

In the present disclosure, the amino acid sequence of SEQ ID NO: 12 may be a sequence of a protein variant in which the 697th amino acid of helicase protein is substituted from histidine (His, H) to tyrosine (Tyr, Y). The helicase protein may be a protein encoded by NCgl1432 gene.

In the present disclosure, the amino acid sequence of SEQ ID NO: 13 may be a sequence of a protein variant in which the 35th amino acid of a protein (hypothetical protein) encoded by NCg11531 gene is substituted from glutamic acid (Glu, E) to lysine (Lys, K).

In the present disclosure, the amino acid sequence of SEQ ID NO: 14 may be a sequence of a protein variant in which the 81th amino acid of a protein (hypothetical protein) encoded by NCgl1588 gene is substituted from valine (Val, V) to isoleucine (lle, I).

In the present disclosure, the amino acid sequence of SEQ ID NO: 15 may be a sequence of a protein variant in which the 75th amino acid of fruR (transcriptional regulator of sugar metabolism) protein is substituted from glycine (Gly, G) to valine (Val, V). The fruR protein may be a protein encoded by NCgl1859 gene.

In the present disclosure, the amino acid sequence of SEQ ID NO: 16 may be a sequence of a protein variant in which the 164th amino acid of miaB ((dimethylallyl)adenosine tRNA methylthiotransferase) protein is substituted from serine (Ser, S) to proline (Pro, P). The miaB protein may be a protein encoded by NCgl1874 gene.

In the present disclosure, the amino acid sequence of SEQ ID NO: 18 may be a sequence of a protein variant of a protein (hypothetical protein) encoded by NCgl2356, in which the 456th amino acid of the protein is substituted from valine (Val, V) to alanine (Ala, A), and the 464th amino acid of the protein is substituted from serine (Ser, S) to proline (Pro, P), and the 465th amino acid of the protein is substituted from alanine (Ala, A) to valine (Val, V), and the 466th amino acid of the protein is substituted from serine (Ser, S) to glycine (Gly, G), and the 496th amino acid of the protein is substituted from glutamic acid (Glu, E) to threonine (Thr, T), and the 499th amino acid of the protein is substituted from serine (Ser, S) to aspartic acid (Asp, D).

The protein variant of the present disclosure may comprise any one amino acid sequence (for example, the amino acid sequence of SEQ ID NO: 13 or SEQ ID NO: 15) selected from the group consisting of amino acid sequences of SEQ ID NO: 1, SEQ ID NO: 3 to SEQ ID NO: 5, SEQ ID NO: 7 to SEQ ID NO: 16, and SEQ ID NO: 18, or comprise an amino acid sequence having homology or identity of at least 70%, 75%, 80%, 85%, 90%, 95%, 96%, 97%, 98%, 99%, 99.5%, 99.7% or 99.9% or more to said amino acid sequence. In addition, it is obvious that even a variant having an amino acid sequence in which some sequences are deleted, modified, substituted, conservatively substituted, or added is included in the scope of the present disclosure, as long as it is an amino acid sequence which has such homology or identity and shows efficacy (efficacy of increasing L-lysine production) corresponding to the variant of the present disclosure.

For example, they are cases of having addition or deletion of a sequence which does not change the function of the variant of the present disclosure, naturally occurring mutation, silent mutation, or conservative substitution at the N-terminus, C-terminus and/or inside the amino acid sequences.

In the protein variant, the conservative substitution, variant, and the like are as described above.

In the present disclosure, the term, 'homology' or 'identity' means the degree of similarity between two given amino acid sequences or nucleotide sequences, and may be expressed as a percentage. The terms, homology and identity may often be used interchangeably.

The sequence homology or identity of a conserved polynucleotide or polypeptide may be determined by a standard alignment algorithm, and a default gap penalty established by the program used may be used together. Substantially, a homologous or identical sequence may be generally hybridized with all or a part of a sequence under medium or high stringent conditions. It is obvious that hybridization also includes hybridization of a polynucleotide with a polynucleotide containing common codons or codons considering codon degeneracy.

Whether any two polynucleotide or polypeptide sequences have homology, similarity, or identity, may be determined using a known computer algorithm such as "FASTA" program using a default parameter such as that in for example, Pearson et al (1988) [Proc. Natl. Acad. Sci. USA 85]: 2444. Otherwise, it may be determined using Needleman-Wunsch algorithm (Needleman and Wunsch, 1970, J. Mol. Biol. 48: 443-453), as performed in Needleman program of EMBOSS package (EMBOSS: The European Molecular Biology Open Software Suite, Rice et al., 2000, Trends Genet. 16: 276-277) (version 5.0.0 or later version) (GCG program package (including Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, the homology, similarity, or identity may be determined using BLAST or ClustalW of National Database Center of Biotechnology Information.

The homology, similarity, or identity of the polynucleotide or polypeptide may be determined by compared sequence information using for example, GAP computer program such as Needleman et al. (1970), J Mol Biol. 48:443, for example, known in Smith and Waterman, Adv. Appl. Math (1981) 2:482. In summary, the GAP program may be defined as a value of dividing a total number of symbols in the shorter of two sequences by the number of similar aligned symbols (i.e., nucleotides or amino acids). The default parameters for the GAP program may comprise (1) a binary comparison matrix (containing values of 1 for identity and 0 for non-identity) and a weighted comparison matrix of Gribskov et al (1986) Nucl. Acids Res. 14: 6745, disclosed in Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a 3.0 penalty for each gap and an additional 0.10 penalty for each symbol in each gap (or gap opening penalty 10, gap extension penalty 0.5); and (3) no penalty for end gaps.

As one example of the present disclosure, the variant of the present disclosure may have activity to increase L-lysine productivity compared to a wild-type polypeptide.

In the present disclosure, the term, "corresponding to" refers to an amino acid residue at a position listed in a polypeptide, or an amino acid residue similar or identical or homologous to a residue listed in a polypeptide. Confirming an amino acid at a corresponding position may determine a specific amino acid of a sequence referring to a specific sequence. The "corresponding region" used in the present disclosure generally refers to a similar or corresponding position in a related protein or reference protein.

For example, any amino acid sequence may be aligned with an amino acid sequence in which the amino acid corresponding to the 75th position from the N-terminus in the amino acid sequence of SEQ ID NO: 105 is substituted with an amino acid (for example, valine) different from the original amino acid, or an amino acid sequence in which the amino acid corresponding to the 35th sequence from the N-terminus in the amino acid sequence of SEQ ID NO: 106 is substituted with an amino acid (for example, lysine) different from the original amino acid; or any one amino acid sequence (for example, the amino acid sequence of SEQ ID NO: 13 or SEQ ID NO: 15) selected from the group consisting of SEQ ID NO: 1, SEQ ID NO: 3 to SEQ ID NO: 5, SEQ ID NO: 7 to SEQ ID NO: 16, and SEQ ID NO: 18, and based on this, each amino acid residue of the amino acid sequence may be numbered with reference to the number position of the amino acid residue corresponding to an amino acid residue of an amino acid sequence in which the amino acid corresponding to the 75th position from the N-terminus in the amino acid sequence of SEQ ID NO: 105 is substituted with an amino acid (for example, valine) different from the original amino acid, or an amino acid sequence in which the amino acid corresponding to the 35th sequence from the N-terminus in the amino acid sequence of SEQ ID NO: 106 is substituted with an amino acid (for example, lysine) different from the original amino acid; or any one amino acid sequence (for example, the amino acid sequence of SEQ ID NO: 13 or SEQ ID NO: 15) selected from the group consisting of SEQ ID NO: 1, SEQ ID NO: 3 to SEQ ID NO: 5, SEQ ID NO: 7 to SEQ ID NO: 16, and SEQ ID NO: 18. For example, the sequence alignment algorithm as described in the present disclosure, may confirm a location of amino acids, or a location where modification such as substitution, insertion or deletion or the like occurs, compared to a query sequence (also called "reference sequence").

For this alignment, for example, Needleman-Wunsch algorithm (Needleman and Wunsch, 1970, J. Mol. Biol. 48: 443-453), Needle program of EMBOSS package (EMBOSS: The European Molecular Biology Open Software Suite, Rice et al., 2000), Trends Genet. 16: 276-277) and the like may be used, but not limited thereto, and a sequence alignment program known in the art, pairwise sequence comparison algorithm and the like may be appropriately used.

Other aspect of the present disclosure provides a polynucleotide encoding the protein variant of the present disclosure.

In the present disclosure, the term, "polynucleotide" refers to a DNA or RNA strand of a certain length or more, which is a polymer of nucleotides in which nucleotide monomers are connected in a long chain form by covalent bonds, and more specifically, a polynucleotide fragment encoding the variant.

In one embodiment, the polynucleotide may be a polynucleotide encoding a protein variant comprising an amino acid sequence in which the amino acid corresponding to the 75th position from the N-terminus in the amino acid sequence of SEQ ID NO: 105 is substituted with an amino acid (for example, valine) different from the original amino acid, or an amino acid sequence in which the amino acid corresponding to the 35th position from the N-terminus in the amino acid sequence of SEQ ID NO: 106 is substituted with an amino acid (for example, lysine) different from the original amino acid.

In one embodiment, the polynucleotide may comprise a polynucleotide comprising any one nucleotide sequence selected from the group consisting of nucleotide sequences of SEQ ID NO: 23, SEQ ID NO: 25 to SEQ ID NO: 27, SEQ ID NO: 29 to SEQ ID NO: 38, and SEQ ID NO: 40, for example, the nucleotide sequence of SEQ ID NO: 37.

The polynucleotide encoding the protein variant of the present disclosure may have or comprise any one nucleotide sequence selected from the group consisting of nucleotide sequences of SEQ ID NO: 23, SEQ ID NO: 25 to SEQ ID NO: 27, SEQ ID NO: 29 to SEQ ID NO: 38, and SEQ ID NO: 40, for example, the nucleotide sequence of SEQ ID NO: 37. In another embodiment, the polynucleotide may comprise a nucleotide sequence having homology or identity of at least 70%, 75%, 80%, 85%, 90%, 95%, 96%, 97%, 98%, 99%, 99.5%, 99.7% or 99.9% or more to the nucleotide sequence. In addition, it is obvious that even a polynucleotide having a nucleotide sequence in which some sequences are deleted, modified, substituted, conservatively substituted, or added is included in the scope of the present disclosure, as long as it is a sequence which has such homology or identity and shows efficacy (efficacy of increasing L-lysine production) corresponding to the variant of the present disclosure.

In one embodiment, the nucleotide sequence or amino acid sequence provided in the present description may include one modified by common mutagenesis, for example, direct evolution and/or site-directed mutagenesis and the like, within a range of maintaining their original functions or desired functions. In one embodiment, that a polynucleotide or polypeptide "comprises a specific nucleotide or amino acid sequence" may mean that the polynucleotide or polypeptide (i) consists of the specific nucleotide sequence or amino acid sequence, or essentially comprises the same, or (ii) consists of an amino acid sequence having homology of 60% or more, 70% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more to the specific nucleotide sequence or amino acid sequence or essentially comprises thereof and maintains the original functions and/or desired functions.

The polynucleotide of the present disclosure may have various modifications in coding regions within a range which does not change the amino acid sequence of the variant of the present disclosure, in consideration of degeneracy of codons or codons preferred in an organism in which the variant of the present disclosure is to be expressed. Specifically, the polynucleotide of the present disclosure may have or comprise a nucleotide sequence with homology or identity of 70% or more, 75% or more, 80% or more, 85% or more, 90% or more, 95% or more, 96% or more, 97% or more, 98% or more, and less than 100% to a nucleotide sequence of a polynucleotide encoding a protein variant comprising the amino acid sequence in which the amino acid corresponding to the 75th position from the N-terminus in the amino acid sequence of SEQ ID NO: 105 is substituted with an amino acid (for example, valine) different from the original amino acid or an amino acid sequence in which the amino acid corresponding to the 35th position from the N-terminus in the amino acid sequence of SEQ ID NO: 106 is substituted with an amino acid (for example, lysine) different from the original amino acid, or any one nucleotide sequence (for example, the nucleotide sequence of SEQ ID NO: 37) selected from the group consisting of nucleotide sequences of SEQ ID NO: 23, SEQ ID NO: 25 to SEQ ID NO: 27, SEQ ID NO: 29 to SEQ ID NO: 38, and SEQ ID NO: 40, or consist of or essentially consist of a nucleotide sequence with homology or identity of 70% or more, 75% or more, 80% or more, 85% or more, 90% or more, 95% or more, 96% or more, 97% or more, 98% or more, and less than 100% to the nucleotide sequence, but not limited thereto.

In addition, the polynucleotide of the present disclosure may comprise a probe that can be prepared from a known gene sequence, for example, any sequence capable of hybridizing with a complementary sequence to all or a part of the polynucleotide sequence of the present disclosure under stringent conditions without limitation. The "stringent condition" means a condition that allows specific hybridization between polynucleotides. For example, a condition of hybridizing polynucleotides with high homology or identity, polynucleotides with homology or identity of 70% or more, 75% or more, 80% or more, 85% or more, 90% or more, 95% or more, 96% or more, 97% or more, 98% or more, or 99% or more, and not hybridizing polynucleotides with homology or identity lower than that, or a condition of washing at a salt concentration and temperature corresponding to 60°C, 1 ×SSC, 0.1% SDS, specifically, 60°C, 0.1×SSC, 0.1% SDS, more specifically, 68°C, 0.1×SSC, 0.1% SDS, which is a washing condition of common southern hybridization, once, specifically, twice to 3 times may be listed.

Hybridization requires that two nucleotides have complementary sequences, but mismatches between bases may be allowed depending on the stringency of hybridization. The term, "complementary" may be used to describe relationship between nucleotide bases capable of hybridizing to each other. For example, in case of DNA, adenosine is complementary to thymine, and cytosine is complementary to guanine. Therefore, the polynucleotide of the present disclosure may also comprise not only a substantially similar nucleotide sequence, but also an isolated nucleic acid fragment complementary to the entire sequence.

Specifically, the polynucleotide with homology or identity to the polynucleotide of the present disclosure may be detected using a hybridization condition including a hybridization step at a Tm value of 55°C and using the afore-mentioned conditions. In addition, the value of Tm may be 60°C, 63°C or 65°C, but not limited thereto, and may be appropriately adjusted by those skilled in the art according to the purpose.

The suitable stringency to hybridize the polynucleotide depends on the length and degree of complementarity of the polynucleotide, and variables are well known in the art.

Other aspect of the present disclosure provides a vector comprising the polynucleotide of the present disclosure. The vector may be an expression vector to express the polynucleotide in a host cell, but not limited thereto.

The vector of the present disclosure may comprise a DNA product comprising a nucleotide sequence of a polynucleotide encoding the target polypeptide operably linked to an expression regulatory region (or expression regulatory sequence) suitable for expressing a target polypeptide in an appropriate host. The expression regulatory region may comprise a promoter that can initiate transcription, any operator sequence to regulate such transcription, a sequence encoding an appropriate mRNA ribosome binding site, and/or a nucleic acid sequence that regulates termination of transcription and translation. The vector may be transformed into a suitable host cell, and then be replicated or function independently of the host genome, and be integrated into the genome itself.

The vector used in the present disclosure is not particularly limited, and any vector known in the art may be used. Examples of the commonly used vector may include plasmids, cosmids, viruses, and bacteriophages in a natural state or a recombined state. For example, as the phage vector or cosmid vector, pWE15, M13, MBL3, MBL4, IXII, ASHII, APII, t10, t11, Charon4A, and Charon21A, and the like may be used, and as the plasmid vector, pDZ-based, pBR-based, pUC-based, pBluescriptll-based, pGEM-based, pTZ-based, pCL-based and pET-based and the like may be used. Specifically, pDZ, pDC, pDCM2, pACYC177, pACYC184, pCL, pECCG117, pUC19, pBR322, pMW118, pCC1BAC, or pDC24 vector, or the like may be used.

As one example, through a vector for inserting chromosome into a cell, a polynucleotide encoding a target polypeptide may be inserted into chromosome. Insertion of the polynucleotide into chromosome may be performed by any method known in the art, for example, homologous recombination, bout not limited thereto. A selection marker to confirm whether the chromosome is inserted may be further comprised. The selection marker is to select a cell transformed with a vector, that is, to confirm whether a target nucleic acid molecule is inserted, and markers which impart selectable phenotypes such as drug resistance, auxotrophy, resistance to a cytotoxic agent, or expression of a surface protein may be used. In an environment treated with a selective agent, only cells expressing the selection marker survive or show other phenotypes, so transformed cells may be selected.

In the present disclosure, the term, "transformation" means introducing a vector comprising a polynucleotide encoding a target polypeptide into a host cell or microorganism so that the polypeptide encoded by the polynucleotide is to be expressed in the host cell. As long as the transformed polynucleotide can be expressed in a host cell, all of them may be included regardless of whether they are inserted into chromosome of the host cell or located outside the chromosome. In addition, the polynucleotide comprises DNA and/or RNA encoding a target polypeptide. The polynucleotide may be introduced in any form, as long as it can be introduced into a host cell and expressed. For example, the polynucleotide may be introduced into a host cell in a form of expression cassette, which is a gene structure comprising all elements required for being expressed by itself. The expression cassette may commonly comprise a promoter operatively linked to the polynucleotide, a transcription termination signal, a ribosome binding site and a translation termination signal. The expression cassette may be in a form of an expression vector capable of self-replication. In addition, the polynucleotide may be introduced into a host cell in its own form and be operatively linked to a sequence required for expression in the host cell, but not limited thereto.

Furthermore, in the above, the term, "operatively linked" refers to that the polynucleotide sequence is functionally linked to a promoter sequence which initiates and mediates transcription of the polynucleotide encoding the target variant of the present disclosure.

Other aspect of the present disclosure provides a microorganism of the genus of *Corynebacterium,* comprising at least one selected from the group consisting of the protein variant of the present disclosure and the polynucleotide of the present disclosure.

The microorganism of the present disclosure may comprise the protein variant of the present disclosure, a polynucleotide encoding the protein variant, or a vector comprising the polynucleotide of the present disclosure.

In the present disclosure, the term, "microorganism (or strain)" includes all of wild-type microorganisms or microorganisms in which genetic modification occurs naturally or artificially, and may be a microorganism of which specific mechanism is weakened or strengthened due to reasons such as insertion of a foreign gene or enhancement or inactivation of activity of an intrinsic gene and the like, which is a microorganism comprising genetic modification for production of a target polypeptide, protein or product.

The microorganism of the present disclosure may be a microorganism comprising any one or more of the protein variant of the present disclosure, the polynucleotide of the present disclosure, and a vector comprising the polynucleotide of the present disclosure; a microorganism modified to express the variant of the present disclosure or the polynucleotide of the present disclosure; a microorganism (for example, recombinant microorganism) expressing the variant of the present disclosure, or the polynucleotide of the present disclosure; or a microorganism (for example, recombinant microorganism) having the activity of the variant of the present disclosure, but not limited thereto.

The microorganism of the present disclosure may be a microorganism having L-lysine productivity.

The microorganism of the present disclosure may be a microorganism naturally having L-lysine productivity, or a microorganism in which the variant of the present disclosure or a polynucleotide encoding thereof (or a vector comprising the polynucleotide) is introduced into a parent strain having no L-lysine productivity and/or L-lysine productivity is imparted, but not limited thereto.

As one example, the microorganism of the present disclosure is a cell or microorganism expressing the protein variant of the present disclosure, as transformed with a vector comprising the polynucleotide of the present disclosure or a polynucleotide encoding the variant of the present disclosure, and for the purpose of the present disclosure, the microorganism of the present disclosure may include all microorganisms capable of producing L-lysine by comprising the variant of the present disclosure. For example, the microorganism of the present disclosure may be a natural wild-type microorganism or a recombinant microorganism with increased L-lysine productivity by introducing the polynucleotide encoding the variant of the present disclosure into a microorganism producing L-lysine.

In one specific embodiment, the microorganism of the present disclosure may have increased L-lysine productivity, compared to a microorganism of the genus of *Corynebacterium* which does not comprise any one selected from the group consisting of the afore-mentioned protein variant of the present disclosure and the polynucleotide encoding the protein variant (that is, a microorganism which does not express any one of a protein variant comprising an amino acid sequence in which the amino acid corresponding to the 75th position from the N-terminus in the amino acid sequence of SEQ ID NO: 105 is substituted with an amino acid (for example, valine) different from the original amino acid, an amino acid sequence in which the amino acid corresponding to the 35th position from the N-terminus in the amino acid sequence of SEQ ID NO: 106 is substituted with an amino acid (for example, lysine) different from the original amino acid; and any one amino acid sequence selected from the group consisting of amino acid sequences of SEQ ID NO: 1, SEQ ID NO: 3 to SEQ ID NO: 5, SEQ ID NO: 7 to SEQ ID NO: 16, and SEQ ID NO: 18, for example, the amino acid sequence of SEQ ID NO: 13 or SEQ ID NO: 15, or a microorganism expressing a wild-type protein corresponding to the protein variant).

The microorganism of the genus of *Corynebacterium* which does not comprise any one selected from the group consisting of the afore-mentioned protein variant of the present disclosure and the polynucleotide encoding the protein variant may be a natural wild-type microorganism or a non-modified microorganism, and may also be expressed as a host cell or parent strain. The microorganism of the genus of *Corynebacterium* which does not comprise any one selected from the group consisting of the afore-mentioned protein variant of the present disclosure and the polynucleotide encoding the protein variant may be specifically ATCC13032 strain and/or CJ3P (US 9556463 B2), but not limited thereto.

The recombinant microorganism with increased L-lysine productivity of the present disclosure may be a microorganism with increased L-lysine productivity compared to a natural wild-type microorganism or a non-modified microorganism, but not limited thereto. As the example, the non-modified microorganism, which is a target strain for comparing the increase in L-lysine productivity, may be ATCC13032 strain and/or CJ3P (US 9556463 B2), but not limited thereto.

In one embodiment, the microorganism with increased L-lysine productivity (produced amount) of the present disclosure may have increased L-lysine productivity (produced amount) of about 1% or more, about 3% or more, about 5% or more, about 7.5% or more, about 10% or more, about 12.5% or more, about 15% or more, about 15.25% or more (the upper limit is not particularly limited, and for example, it may be about 200% or less, about 150% or less, about 100% or less, or about 50% or less, about 30% or less), compared to a parent strain before mutated or non-modified microorganism, but is not limited thereto. In another embodiment, the microorganism with increased L-lysine productivity (produced amount) of the present disclosure may have increased L-lysine productivity (produced amount) of about 1.01 times or more, about 1.03 times or more, about 1.05 times or more, about 1.075 times or more, about 1.1 times or more, about 1.125 times or more, about 1.15 times or more, about 1.1525 times or more (the upper limit is not particularly limited, and for example, it may be about 10 times or less, about 5 times or less, about 3 times or less, or about 2 times or less), compared to a parent strain before mutated or non-modified microorganism, but is not limited thereto.

The term "about" is a range including all of ±0.5, ±0.4, ±0.3, ±0.2, ±0.1, and the like, and includes all numerical values identical or similar to the numerical value appearing after the term of about, but is not limited thereto.

In the present disclosure, the term, "non-modified microorganism" does not exclude a microorganism comprising mutation that can occur naturally in a microorganism, and may mean a wild-type microorganism or natural microorganism itself, or a microorganism before traits are changed due to genetic mutation caused by natural or artificial factors. For example, the non-modified microorganism may mean a microorganism in which the protein variant described in the present description is not introduced or before introduced. The "non-modified microorganism" may be interchangeably used with "strain before modification", "microorganism before modification", "non-mutated strain", "non-modified strain", "non-mutated microorganism" or "reference microorganism".

In one embodiment, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* and more specifically, it may be *Corynebacterium glutamicum.*

In the present disclosure, the term, "weakening" of the polypeptide, is a concept including both reducing activity or having no activity compared to the intrinsic activity. The weakening may be interchangeably used with terms such as inactivation, deficiency, down-regulation, decrease, reduce, attenuation, and the like.

The weakening may also include cases where the activity of the polypeptide itself is reduced or removed compared to activity of the polypeptide possessed by the original microorganism due to mutation of a polynucleotide encoding the polypeptide and the like, cases where the overall polypeptide activity level and/or concentration (expression level) is lower than a natural strain in cells due to inhibition of expression or inhibition of translation into a polypeptide of a gene of a polynucleotide encoding the same, and the like, cases where expression of the polynucleotide is not done at all, and/or cases where there is no activity of the polypeptide even if the polynucleotide is expressed. The "intrinsic activity" refers to activity of a specific polypeptide originally possessed by a parent strain before trait changes or non-modified microorganism, when traits are changed due to genetic mutation caused by natural or artificial factors. This may be used interchangeably with "activity before modification". That activity of a polypeptide is "inactivated, deficient, decreased, down-regulated, reduced, or attenuated" compared to the intrinsic activity, refers to that it is lowered compared to the activity of a specific polypeptide originally possessed by a parent strain before trait changes or non-modified microorganism.

This weakening of the activity of the polypeptide may be performed by any method known in the art, but not limited thereto, and may be achieved by application of various methods well known in the art (for example, Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the polypeptide may be
1) deficiency of the entire or a part of a gene encoding a polypeptide;
2) modification of an expression regulatory region (or expression regulatory sequence) so that expression of a gene encoding a polypeptide is reduced;
3) modification (for example, deletion/substitution/addition of at least one amino acid in the amino acid sequence) of an amino acid sequence composing the polypeptide so that the activity of the polypeptide is removed or weakened;
4) modification (for example, addition/substitution/addition of at least one nucleic acid base in the nucleic acid base sequence of the polypeptide gene to encode a polypeptide modified so that the activity of the polypeptide is removed or weakened) of a gene sequence encoding the polypeptide so that the activity of the polypeptide is removed or weakened;
5) modification of a base sequence encoding an initiation codon or 5'-UTR area of gene transcriptome encoding a polypeptide;
6) introduction of an antisense oligonucleotide (for example, antisense RNA) complementarily binding to transcriptome of a gene encoding a polypeptide;
7) addition of a sequence complementary to Shine-Dalgarno sequence in front of the Shine-Dalgarno sequence of a gene encoding a polypeptide so as to form a secondary structure incapable of attachment of ribosome;
8) addition of a promoter transcribed in the opposite direction at the 3' end of an ORF (open reading frame) of a gene sequence encoding a polypeptide (Reverse transcription engineering, RTE); or
9) adjustment of cellular localization of a protein (polypeptide); or
10) a combination of at least 2 selected from the 1) to 9), but not particularly limited thereto.

For example,
the 1) deficiency of the entire or a part of a gene encoding a polypeptide may be removal of the entire polynucleotide encoding an intrinsic target polypeptide in chromosome, replacement with a polynucleotide in which some nucleotides are deleted, or replacement with a marker gene.

In addition, the 2) modification of an expression regulatory region (or expression regulatory sequence) may be mutation occurrence on the expression regulatory region (or expression regulatory sequence) by deletion, insertion, non-conservative or conservative substitution or a combination thereof, or replacement with a sequence having weaker activity. The expression regulatory region includes a promoter, an operator sequence, a sequence encoding a ribosome binding site, and a sequence regulating termination of transcription and translation, but not limited thereto.

Furthermore, the 3) modification of a base sequence encoding an initiation codon or 5'-UTR area of gene transcriptome encoding a polypeptide may be for example, substituting it with a base sequence encoding another initiation codon with a lower polypeptide expression rate compared to the intrinsic initiation codon, but not limited thereto.

Moreover, the modification of an amino acid sequence or polynucleotide sequence of the 4) and 5) may be occurrence of mutation in the sequence by deletion, insertion, non-conservative or conservative substitution or a combination thereof, or replacement with an amino acid sequence or polynucleotide sequence improved to have weaker activity or an amino acid sequence or polynucleotide sequence improved to have no activity, but not limited thereto. For example, by forming a stop codon by introducing mutation in a polynucleotide sequence, expression of a gene may be inhibited or weakened, but not limited thereto.

The 6) introduction of an antisense oligonucleotide (for example, antisense RNA) complementarily binding to transcriptome of a gene encoding a polypeptide may refer to for example, the document [Weintraub, H. et al., Antisense-RNA as a molecular tool for genetic analysis, Reviews - Trends in Genetics, Vol. 1(1) 1986].

The 7) addition of a sequence complementary to Shine-Dalgarno sequence in front of the Shine-Dalgarno sequence of a gene encoding a polypeptide so as to form a secondary structure incapable of attachment of ribosome may make mRNA translation impossible or inhibit its speed.

The 8) addition of a promoter transcribed in the opposite direction at the 3' end of an ORF (open reading frame) of a gene sequence encoding a polypeptide (Reverse transcription engineering, RTE) may create an antisense nucleotide complementary to the transcriptome of the gene encoding the polypeptide to weaken the activity.

The 9) adjustment of cellular localization of a protein (polypeptide) may target a protein (polypeptide) to an intracellular specific organelle or specific intracellular space. For example, by addition or removal of a leader sequence functioning in targeting a protein (polypeptide), it may target it to periplasm or cytoplasm, but not limited thereto.

Such weakening of the activity of the polypeptide, may weaken the activity or concentration expression level of the corresponding polypeptide based on the activity or concentration of the expressed polypeptide in a wild-type or pre-transformed microbial strain, or increasing the amount of products produced from the corresponding polypeptide, but is not limited thereto.

In the present disclosure, the term, "enhancement" of the polypeptide activity, means that the activity of the polypeptide is increased compared to the intrinsic activity. The enhancement may be interchangeably used with terms such as activation, up-regulation, overexpression, increase and the like. Herein, the activation, up-regulation, overexpression, and increase may include showing activity which is not originally present, or showing activity improved compared to the intrinsic activity or activity before modification. The "intrinsic activity" refers to activity of a specific polypeptide originally possessed by a parent strain before trait changes or non-modified microorganism, when traits are changed due to genetic mutation caused by natural or artificial factors. This may be used interchangeably with "activity before modification". That activity of a polypeptide "is enhanced", "is up-regulated", "is overexpressed" or "increases" compared to the intrinsic activity, refers to that it is improved compared to the activity and/or concentration (expression level) of a specific polypeptide originally possessed by a parent strain before trait changes or non-modified microorganism.

The enhancement may be achieved by introducing a foreign polypeptide, or enhancement of activity of an intrinsic polypeptide and/or concentration (expression level). Whether the activity of the polypeptide is enhanced may be confirmed by the degree of activity, expression level of the corresponding polypeptide, or an increase of the amount of products released from the corresponding polypeptide.

For the enhancement of the activity of the polypeptide, various methods well known in the art may be applied, and there is no limitation, as long as the activity of a target polypeptide can be enhanced compared to a microorganism before modification. Specifically, genetic engineering and/or protein engineering well known to those skilled in the art, which are routine methods of molecular biology, may be used, but not limited thereto (for example, Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be
1) an increase of a copy number in cells of a polynucleotide encoding a polypeptide;
2) replacement of a gene expression regulatory region on chromosome encoding a polypeptide with a sequence having strong activity;
3) modification of a base sequence encoding an initiation codon or 5'-UTR area of gene transcriptome encoding a polypeptide;
4) modification of an amino acid sequence of the polypeptide so that the activity of the polypeptide is enhanced;
5) modification of a polynucleotide sequence encoding the polypeptide so that the activity of the polypeptide is enhanced (for example, modification of a polynucleotide of the polypeptide gene so as to encode a polypeptide modified to enhance the activity of the polypeptide);
6) introduction of a foreign polypeptide showing activity of a polypeptide or a foreign polynucleotide encoding the same;
7) codon optimization of a polynucleotide encoding a polypeptide;
8) modification or chemical modification by analyzing the tertiary structure of a polypeptide and selecting an exposed site;
9) adjustment of cellular localization of a protein (polypeptide); or
10) a combination of at least 2 selected from the 1) to 9), but not particularly limited thereto.

The 1) increase of a copy number in cells of a polynucleotide encoding a polypeptide may be achieved by introduction of a vector which can replicate and function regardless of a host, in which a polynucleotide encoding the corresponding polypeptide is operatively linked. Otherwise, it may be achieved by introduction of 1 copy or 2 copies or more of the polynucleotide encoding the corresponding polypeptide into chromosome in a host cell. The introduction into the chromosome may be performed by introducing a vector capable of inserting the polynucleotide into chromosome of the host cell into the host cell, but not limited thereto. The vector is as described above.

The 2) replacement of a gene expression regulatory region (or expression regulatory sequence) on chromosome encoding a polypeptide with a sequence having strong activity, may be for example, occurrence of mutation in the sequence by deletion, insertion, non-conservative or conservative substitution or a combination thereof so as to further intensify activity of the expression regulatory region, or replacement with a sequence having stronger activity. The expression regulatory region is not particularly limited thereto, but may comprise a promoter, an operator sequence, a sequence encoding a ribosome binding site, and a sequence regulating termination of transcription and translation and the like. As one example, the original promoter may be replaced with a strong promoter, but not limited thereto.

Examples of the known strong promoter include CJ1 to CJ7 promoters (U.S. Patent US 7662943 B2), lac promoter, trp promoter, trc promoter, tac promoter, lambda phage PR promoter, PL promoter, tet promoter, gapA promoter, SPL7 promoter, SPL13(sm3) promoter (U.S. Patent US 10584338 B2), O2 promoter (U.S. Patent US 10273491 B2), tkt promoter, yccA promoter, and the like, but not limited thereto.

The 3) modification of a base sequence encoding an initiation codon or 5'-UTR area of gene transcriptome encoding a polypeptide may be for example, substituting it with a base sequence encoding another initiation codon with a higher polypeptide expression rate compared to the intrinsic initiation codon, but not limited thereto.

The modification of an amino acid sequence or polynucleotide sequence of the 4) and 5) may be occurrence of mutation in the sequence by deletion, insertion, non-conservative or conservative substitution or a combination thereof, or replacement with an amino acid sequence or polynucleotide sequence improved to have stronger activity for the amino acid sequence of the polypeptide or a polynucleotide sequence encoding the polypeptide so as to strengthen the activity of the polypeptide, but not limited thereto. The replacement may be performed specifically by inserting a polynucleotide into chromosome by homologous recombination, but not limited thereto. The vector used then may further comprise a selection marker for confirming chromosome insertion. The selection marker is as described above.

The 6) introduction of a foreign polypeptide showing activity of a polypeptide may be introduction of a foreign polynucleotide encoding a polypeptide showing the same/similar activity to the polypeptide into a host cell. The foreign polynucleotide is not limited in its origin or sequence as long as it shows the same/similar activity to the polypeptide. The method used for the introduction may be performed by those skilled in the art by appropriately selecting a known transformation method, and by expressing the introduced polynucleotide in a host cell, a polypeptide may be produced and its activity may be increased.

The 7) codon optimization of a polynucleotide encoding a polypeptide may be codon optimization so that transcription or translation of an intrinsic polynucleotide is increased in a host cell, or codon optimization so that a foreign polynucleotide is optimized in a host cell to achieve transcription and translation.

The 8) modification or chemical modification by analyzing the tertiary structure of a polypeptide and selecting an exposed site may determine template protein candidates depending on the degree of similarity of sequences by comparing sequence information of a polypeptide to be analyzed, and confirm the structure based on this, and select an exposed site to be modified or chemically modified, and transform or modify it.

The 9) adjustment of cellular localization of a protein (polypeptide) may target a protein (polypeptide) into an intracellular specific organelle or specific intracellular space. For example, by addition or removal of a leader sequence functioning in targeting a protein (polypeptide), it may target it to periplasm or cytoplasm, but not limited thereto.

Such enhancement of the polypeptide activity means increasing activity or concentration expression level of the corresponding polypeptide based on the activity or concentration of the expressed polypeptide in a wild-type or pre-transformed microbial strain, or increasing the amount of products produced from the corresponding polypeptide, but is not limited thereto.

In the microorganism of the present disclosure, modification of a part or all of a polynucleotide (for example, modification to encode the afore-mentioned protein variant) may be induced by (a) homologous recombination using a vector for inserting chromosome into a microorganism or genome editing using genetic scissors (engineered nuclease, e.g., CRISPR-Cas9) and/or (b) treatment of light such as ultraviolet rays and radioactive rays and the like and/or chemical substances, but not limited thereto. The method of modifying a part of all of the gene may include a method for DNA recombination technology. For example, by injecting a nucleotide sequence or vector comprising a nucleotide sequence homologous to a target gene into the microorganism to cause homologous recombination, deletion of a part or all of the gene may be achieved. The injected nucleotide sequence or vector may include a dominant selection marker, but is not limited thereto.

In the microorganism of the present disclosure, the protein variant, polynucleotide and L-lysine and the like are as described in other aspects above.

Other aspect of the present disclosure provides a method of producing L-lysine, comprising culturing a microorganism of the genus of *Corynebacterium* comprising at least one selected from the group consisting of the protein variant of the present disclosure and the polynucleotide encoding the protein variant in a medium.

The method of producing L-lysine of the present disclosure may comprise culturing a microorganism of the genus of Corynebacterium comprising the protein variant of the present disclosure or the polynucleotide of the present disclosure or the vector of the present disclosure in a medium.

In the present disclosure, "culturing" means growing a microorganism of the genus of *Corynebacterium* of the present disclosure under an appropriately adjusted environmental condition. The culturing process of the present disclosure may be conducted according to an appropriate medium and culturing conditions known in the art. This culturing process may be easily adjusted and used by those skilled in the art depending on the selected strain. Specifically, the culturing may be batch, continuous, and/or fed-batch, but not limited thereto.

In the present disclosure, the term "medium" refers to a material in which nutrients required for culturing the microorganism of the genus of *Corynebacterium* are mixed as main components, and supplies nutrients and growth factors and the like including water which is essential for survival and growth. Specifically, the medium used for culturing the microorganism of the genus of *Corynebacterium* of the present disclosure and other culturing conditions may be used as long as it is a medium used for culturing a common microorganism without particular limitation, but the microorganism of the genus of *Corynebacterium* of the present disclosure may be cultured in a common medium containing a suitable carbon source, a nitrogen source, a phosphorus source, an inorganic compound, an amino acid and/or a vitamin and the like by adjusting temperature, pH and the like under an aerobic condition.

Specifically, the culture medium for the microorganism of the genus of *Corynebacterium* may be found in the document ["Manual of Methods for General Bacteriology" by the American Society for Bacteriology (Washington D.C., USA, 1981)].

In the present disclosure, as the carbon source, carbohydrates such as glucose, saccharose, lactose, fructose, sucrose, maltose, etc.; sugar alcohols such as mannitol, sorbitol, etc., organic acids such as pyruvate, lactate, citrate, etc.; amino acids such as glutamic acid, methionine, lysine, etc. and the like, may be included. In addition, natural organic nutrients such as starch hydrates, molasses, blackstrap molasses, rice bran, cassava, sugar cane residues, and corn steep liquor may be used, and specifically, carbohydrates such as glucose and sterilized pre-treated molasses (that is, molasses converted with reducing sugars) and the like may be used, and other carbon sources in an appropriate amount may be variously used without limitation. These carbon sources may be used alone or at least two kinds may be used in combination, but not limited thereto.

As the nitrogen source, inorganic nitrogen sources such as ammonia, ammonium sulfate, ammonium chloride, ammonium citrate, ammonium phosphate, ammonium carbonate, ammonium nitrate and the like; and organic nitrogen sources such as amino acids including glutamic acid, methionine, glutamine and the like, peptone, NZ-amine, meat extract, yeast extract, malt extract, corn steep liquor, casein hydrates, fish or degraded products thereof, defatted soybean cake or degraded products thereof, and the like may be used. These nitrogen sources may be used alone or at least two may be used in combination, but not limited thereto.

As the phosphorus source, potassium phosphate monobasic, potassium phosphate dibasic, or sodium-containing salts corresponding thereto, or the like may be included. As the inorganic compound, sodium chloride, calcium chloride, iron chloride, magnesium sulfate, iron sulfate, manganese sulfate, calcium carbonate and the like may be used, and in addition thereto, amino acids, vitamins, and/or appropriate precursors and the like may be included. These components or precursors may be added in a batch or continuous method. However, it is not limited thereto.

In addition, by adding a compound such as ammonium hydroxide, potassium hydroxide, ammonia, phosphoric acid, sulfuric acid and the like to a medium during culturing of the microorganism of the genus of Corynebacterium of the present disclosure by an appropriate method, the pH of the medium may be adjusted. Furthermore, during culturing, foam generation may be inhibited using an antifoaming agent such as fatty acid polyglycol ester. Moreover, in order to maintain the aerobic state of the medium, oxygen or oxygen-containing gas may be injected into the medium, or in order to maintain the anaerobic and microaerobic state, gas may be not injected or nitrogen, hydrogen or carbon dioxide gas may be injected, but not limited thereto. In one embodiment, the method of producing L-lysine may be performed at pH 6 to pH 9, and more specifically, it may be performed at pH 6 to pH 9, pH 6 to pH 8.5, pH 6 to pH 8, pH 6 to pH 7.5, pH 6 to pH 7.25, pH 6 to pH 7, pH 6.5 to pH 9, pH 6.5 to pH 8.5, pH 6.5 to pH 8, pH 6.5 to pH 7.5, pH 6.5 to pH 7.25, pH 6.5 to pH 7, pH 6.75 to pH 9, pH 6.75 to pH 8.5, pH 6.75 to pH 8, pH 6.75 to pH 7.5, pH 6.75 to pH 7.25, or pH 6.75 to pH 7, but not limited thereto.

The culture temperature in the culturing of the present disclosure may be maintained at 20 to 45°C, specifically, at 25°C to 40°C, 25°C to 40°C, 25°C to 37°C, 25°C to 35°C, 27°C to 40°C, 27°C to 37°C, 27°C to 35°C, 30°C to 40°C, 30°C to 37°C, or 30°C to 35°C, but not limited thereto. In the culturing of the present disclosure, the time of culturing may be about 10 to 160 hours, but not limited thereto.

The L-lysine produced by the culturing of the present disclosure may be released to a medium or remain in cells.

The method of producing L-lysine of the present disclosure, may further comprise preparing the microorganism of the genus of *Corynebacterium* of the present disclosure, preparing a medium for culturing the strain, or a combination thereof (in any order), for example, before the culturing.

The method of producing L-lysine of the present disclosure, may further comprise recovering L-lysine from the medium according to the culturing (medium in which culturing is performed) or the microorganism of the genus of *Corynebacterium.* The recovering may be further comprised after the culturing.

The recovering may collect targeted L-lysine using an appropriate method known in the art according to the culturing method of the microorganism of the present disclosure, for example, a batch, continuous or fed-batch culturing method, or the like. For example, centrifugation, filtration, treatment by a crystallized protein precipitator (salting out), extraction, ultrasonic crushing, ultrafiltration, dialysis, various kinds of chromatography such as molecular sieve chromatography (gel filtration), adsorption chromatography, ion exchange chromatography, affinity chromatography, and the like, HPLC or a combination of these methods may be used, and using an appropriate method known in the art, targeted L-amino acids may be recovered from a medium or microorganism.

In addition, the method of producing L-lysine of the present disclosure may additionally comprise a purification step. The purification may be performed, using an appropriate method known in the art. In one embodiment, when the method of producing L-lysine of the present disclosure comprises both the recovering step and purification step, the recovering step and purification step may be continuously or non-continuously performed regardless of the order, or may be performed by being integrated as one step, but not limited thereto.

In the method of the present disclosure, the variant, polynucleotide, vector and strain and the like are described in other aspects above.

Other aspect of the present disclosure provides a composition for producing L-lysine comprising at least one selected from the group consisting of the protein variant of the present disclosure, a polynucleotide encoding the protein variant, a vector comprising the polynucleotide, the afore-mentioned microorganism of the present disclosure, and a medium in which this is cultured.

The composition of the present disclosure may further comprise any appropriate excipient commonly used for compositions for producing amino acids, and such an excipient, may be for example, a preservative, wetting agent, dispersant, suspending agent, buffer, stabilizer, or tonicifying agent, or the like, but not limited thereto.

In the composition of the present disclosure, the variant, polynucleotide, vector, strain, medium and L-amino acid and the like are as described in other aspects above.

According to other aspect of the present disclosure, the present disclosure may provide a method of increasing L-lysine productivity of the microorganism, a method of imparting L-lysine productivity to the microorganism, and/or a method of preparing a microorganism with increased L-lysine productivity, comprising introducing (for example, transforming) the afore-mentioned protein variant of the present disclosure, a polynucleotide encoding the variant, and/or a vector comprising the polynucleotide into a microorganism.

**In** the method of increasing L-lysine productivity of the microorganism, the method of imparting L-lysine productivity to the microorganism, and/or the method of preparing a microorganism with increased L-lysine productivity, the polynucleotide, recombinant vector, and microorganism and the like are as described above.

According to other aspect of the present disclosure, the present disclosure provides a use for producing L-lysine, and/or preparing a microorganism producing L-lysine, and/or imparting and/or increasing L-lysine productivity of a microorganism, of at least one selected from the group consisting of the afore-mentioned protein variant; the polynucleotide encoding the protein variant; the recombinant vector comprising the polynucleotide; and the microorganism comprising the protein variant, the polynucleotide encoding the protein variant, and/or the recombinant vector comprising the polynucleotide.

In the use of producing L-lysine, and/or preparing a microorganism producing L-lysine, and/or imparting and/or increasing L-lysine productivity of a microorganism, the protein variant, polynucleotide, recombinant vector, and microorganism, and the like are described above.

According to other aspect of the present disclosure, the present disclosure provides a composition, method, product, process, or use characterized by at least one element disclosed in the present disclosure.

### [ADVANTAGEOUS EFFECTS]

When culturing a microorganism of the genus of Corynebacterium, comprising the present variant of the present disclosure, high yield L-lysine production is possible compared to a microorganism having a conventional non-modified polypeptide.

### [MODE FOR INVENTION]

Hereinafter, the present disclosure will be described in more detail by examples. These examples are intended to more specifically describe the present disclosure only, and it will be obvious to those skilled in the art that the scope of the present disclosure is not limited by these examples, according to the gist of the present disclosure.

### Examples

(Throughout the present description, "%" used to indicate the concentration of a specific substance, unless otherwise mentioned, is (weight/weight) % for solid/solid, (weight/volume) % for solid/liquid, and (volume/volume) % for liquid/liquid.)

### Example 1: Selection of mutant strain with increased lysine productivity by artificial mutation

### Example 1-1: Random mutagenesis through UV irradiation

To select a mutant strain with increased lysine productivity, a lysine producing strain, CJ3P (US 9556463 B2) was smeared on a nutrient medium comprising agar and cultured at 30°C for 16 hours. Colonies obtained in this way were irradiated with UV (Ultraviolet mutation) at a room temperature and random mutation was induced on the genome in the strain. The composition of the nutrient medium is as follows.

### <Nutrient medium (pH 7.2)>

Glucose 10g, meat extract 5g, polypeptone 10g, sodium chloride 2.5g, yeast extract 5g, agar 20g, urea 2g (on the basis of 1 liter of distilled water)

### Example 1-2: Selection of strain with improved L-lysine productivity

The secured colonies of about 3,000 were inoculated in a selection medium of 300 *µ*ℓ, respectively, and cultured in a 96-deep well plate at 32 °C, 1000 rpm for about 24 hours. In order to analyze the production amount of the produced L-amino acids in the cultured solution, the ninhydrin method was used (J. Biol. Chem. 1948. 176:367-388). After completing the culturing, 10 *µ*ℓ of the culture supernatant and 190 *µ*ℓ of the ninhydrin reaction solution were reacted at 65 °C for 30 minutes, and then, the absorbance was measured with a spectrophotometer at a wavelength of 570 nm and about 30 colonies of the mutant strains showing an absorbance increased by 10% or more, compared to the absorbance of the control CJ3P strain, were selected. Other colonies showed similar or reduced absorbance compared to the control group. The composition of the selection medium is as follows.

### <Selection medium (pH 8.0)>

Glucose 10 g, ammonium sulfate 5.5 g, magnesium sulfate heptahydrate 1.2 g, potassium phosphate monobasic 0.8 g, potassium phosphate dibasic 16.4 g, biotin 100 *µ*g, thiamine-HCl 1000 *µ*g, calcium pantothenate 2000 *µ*g, nicotinamide 2000 *µ*g (on the basis of I liter of distilled water)

For the selected 30 strains, the ninhydrin reaction was repeatedly performed by the same method, and top 10 kinds of the strains with improved L-lysine productivity compared to the CJ3P strain were selected.

### Example 1-3: Analysis of L-lysine productivity of selected mutant strains

In order to finally select strains with increased L-lysine productivity for the 10 kinds of strains selected in Example 1-2, a reproducibility test was conducted in a flask using the following mediums.

The 10 kinds of the strains and a parent strain were inoculated in a 250 ml corner-baffled flask containing 25 ml of seed medium, respectively, and cultured with shaking at 200 rpm at 30°C for 20 hours. The seed cultured solution of 1 ml was inoculated in a 250 ml corner-baffled flask containing 24 ml of production medium, and cultured with shaking at 200 rpm at 30°C for 72 hours. The compositions of the seed medium and production medium are as follows.

### <Seed medium (pH 7.0)>

Glucose 20 g, peptone 10 g, yeast extract 5 g, urea 1.5 g, KH₂PO₄ 4 g, K₂HPO₄ 8g, MgSO₄·7H₂O 0.5 g, biotin 0.1 mg, thiamine HCl 1 mg, calcium-pantothenate 2 mg, nicotinamide 2 mg (on the basis of 1 liter of distilled water)

### <Production medium (pH 7.0)>

Glucose 100 g, (NH₄)₂SO₄ 40 g, soybean protein 2.5 g, corn steep solids 5 g, urea 3 g, KH₂PO₄ 1 g, MgSO₄·7H₂O 0.5 g, biotin 100 *µ*g, thiamine hydrochloride 1000 *µ*g, calcium-pantothenate 2000 *µ*g, nicotinamide 3000 *µ*g, CaCO₃ 30 g (on the basis of 1 liter of distilled water)

After completing the culturing, the L-lysine concentration in the cultured solution was analyzed using high-performance liquid chromatography (HPLC), and the L-lysine production concentration of each mutant strain was shown in Table 1 below.

**[Table 1]**

| strain | L-lysine (g/L) |
|---|---|
| CJ3P | 7.2 |
| CJ3P_mt1 | 7.8 |
| CJ3P_mt2 | 7.9 |
| CJ3P_mt3 | 7.5 |
| CJ3P_mt4 | 8.1 |
| **CJ3P_mt5** | **8.8** |
| CJ3P_mt6 | 8.0 |
| CJ3P_mt7 | 7.9 |
| CJ3P_mt8 | 7.8 |
| CJ3P_mt9 | 7.8 |
| CJ3P_mt10 | 7.6 |

Among the selected 10 kinds of mutant strains, CJ3P_mt5 was finally selected as a strain with meaningfully improved L-lysine productivity.

### Example 2. Confirmation of mutation by whole-genome sequencing (WGS)

For the selected CJ3P_mt5 strain in Example 1, whole-genome sequencing (WGS) was conducted, and it was compared with the parent strain, CJ3P, thereby confirming genes with changes in the protein sequence due to nucleotide sequence mutation, and the amino acid sequences of the protein variants and the nucleotide sequences of the genes encoding the protein variants were described in Table 2 and Table 3.

**[Table 2]**

| amin o acid of wild-type strai n | Protei n mutati on locati on | amino acid of variant strain | gene ID | Gene Name | Expressed protein | SEQ ID NO of the variant amino acid sequen ce | SEQ ID NO of the variant nucleoti de sequenc e |
|---|---|---|---|---|---|---|---|
| N | 184 | I | NCgl00 30 | | ABC transporter permease | 1 | 23 |
| E | 418 | G | NCgl01 79 | | Transposase | 2 | 24 |
| A | 86 | V | NCgl02 88 | nth | Endolll-related endonuclease | 3 | 25 |
| E | 363 | D | NCgl03 37 | wzz | chromosome partitioning | 4 | 26 |
| | | | | | ATPase | | |
| N | 210 | D | NCgl04 58 | nusG | transcription antitermination protein NusG | 5 | 27 |
| E | 189 | D | NCgl06 27 | | hypothetical protein | 6 | 28 |
| V | 23 | A | NCgl06 86 | | hypothetical protein | 7 | 29 |
| L | 1112 | F | NCgl07 06 | | Type II restriction enzyme, methylase subunits | 8 | 30 |
| W | 101 | R | NCgl07 99 | mctC | Na+/proline, Na+/panthothe nate symporter or related permease | 9 | 31 |
| D | 461 | E | NCgl11 65 | atpD | ATP synthase F0F1 subunit beta | 10 | 32 |
| G | 456 | D | NCgl12 62 | leuC | isopropylmalate isomerase large subunit | 11 | 33 |
| H | 697 | Y | NCgl14 32 | | helicase | 12 | 34 |
| E | 35 | K | NCgl15 31 | ribX | hypothetical protein | 13 | 35 |
| V | 81 | I | NCgl15 88 | | hypothetical protein | 14 | 36 |
| G | 75 | V | NCgl18 59 | fruR | transcriptional regulator of sugar metabolism | 15 | 37 |
| S | 164 | P | NCgl18 74 | miaB | (dimethylallyl)ad enosine tRNA methylthiotransf erase | 16 | 38 |
| C | 33 | S | NCgl30 69 | | hypothetical protein | 17 | 39 |
| V | 456 | A | NCgl23 56 | | hypothetical protein | 18 | 40 |
| S | 464 | P | NCgl23 56 | | | | |
| A | 465 | V | NCgl23 56 | | | | |
| S | 466 | G | NCgl23 56 | | | | |
| E | 496 | T | NCgl23 56 | | | | |
| S | 499 | D | NCgl23 56 | | | | |
| P | 39 | R | NCgl30 82 | | hypothetical protein | 19 | 41 |
| T | 303 | S | NCgl25 42 | | transposase | 20 | 42 |
| P | 377 | S | NCgl27 65 | pck | phosphoenolpyr uvate carboxykinase | 21 | 43 |
| F | 220 | C | NCgl28 16 | | integral membrane transport protein | 22 | 44 |

**[Table 3]**

| SE Q ID N O: | Sequence (amino acid sequence: N-terminal to C-terminal; nucleotide sequence: 5'→3') |
|---|---|
| 1 | MTKIKSGEASTSIVERALKRPELTSLLGAVLVFTLFMVVAPAFRSWDSMATVLYASSTIGIM AVAVGLLMIADEFDLSTGVAVTTAALAASMFSYNLWLNTWVGALIALVISLAIGFFNGFLV VKTKIASFLITLATFLMLQGINLAVTKLISGTVATPTIADMEGFPSARAVFASSIPIFGVIIRITV FWWLLFVIVGTFVLFKTRIGNWIFAVGGDEEAARAVGVPVRGVKIGLFMFVGFAAWFVG MHNLFLFDSIQAGQGVGNEFLYIIAAVIGGISMTGGRGTVVGTMIGALIFGMTNQGIVYA GWNPDWFMFFLGGTLLLAVLLNHRFERFNKERS |
| 2 | MHHEQPEGCEVGIRRTIPEESRTAFLDMINQGMSGLAASTAVGVSEFTGRKWAKAAGVK LTRGPRGGNAFDTAEKLEIAASMLEKGCLPREIGEYVGMTRANISLWRKQGPDKLRQRAA TLRTGKRAAEFIHAPVMGPYYGPRTLHQVLREDYTTLFDELSALGLPAQVCGALLHLAPPP SLRFSYMSCVVPLFADEIKVVGQGTRLSLEEKMMIQRFHDTGVSAAEIGRRLGRCRQTISR ELRRGQDDDGRYRARDSYEGAIRKLARPKTPKLDANRRLRAVVVEALNNKLSPEQISGLL ATEHANDSSMQISHETIYQALYVQGKGALRDELKVEKFLRTGRKGRKPQSKLPSRGKPWV EGALISQRPAEVADRAVPGHWEGDLVIGGENQATALVTLVERTSRLTLIKRLGVNHGAST VTDALVEMMGDLPQALRRSLTWDQGVEMAEHARFSVVTKCPVFFCDPHSPWQRGSNE NTNGLVRDFFPKGTNFAKVSDEEVQRAQDLLNYRPRKMHGFKSATQVYEKIVVGASTD |
| 3 | MGSITPQKRPRVGSHIANKGQETDIGRKRRARRINRTLTVAYPDAHCELDFTNPLELTVATI LSAQCTDVRVNQVTPALFKRYPTVTDYANADRTELEEFIRPTGFYRNKATSLIGLGEALISL HDGQVPGTLEQLVELPGVGRKTANVVLGNAFGVPGITVDTHFGRLVRRLKLTDEEDPVKV EKVMNELIEKPEWTMFSHRLIFHGRRICHSRRAACGACMLAADCPSFGLEGPSDPFEAQK LIKSDDREHLLKMAGM |
| 4 | MELREYATILMKNWVLIVIASILGIAAGAGFSLLATPEYQSRTQLYVSVRSGAGTTSDMVQ GANFSRQIVNSYVDVIKTGVVLEPVVDELGLELTANQLSSHISAASPADTALINITASSPSP QQAAEIANAVGESFKNVIQTELEPDSGDGMSPINLTTTQVALEPSSPVSPNVMMNILLGL LVGLAIGVGIAVLRAALDTRIHSLRDIEEVTDKPLLGGIIADSEVEKHPLIIKHKPHSPIAESFR ALRTNLQFLNVGGSSSVFVISSANPGEGKSTTSVNLALALAEAGSRVALIEADLRLPRVSKY LGVEGNAGLTDILIGKAEVNDVLQRWGRTQLYYLPAGRIPPNPSELLGSAEMDKVIAELEE SFDYVIIDAPPALAVTDAAVIGHGKAGILIAVSAGSTKKPELEATLSTLENADANVVGVVAT MLPPKSVAGYGYGNYGYGDTSKINAPKPDNTELTTTDASKANNEQ |
| 5 | MSDENINEFEQDEDLNFGASFSDEFADDDFDAEADVEADAAAEASALEAEQDLEEETLD APEEAAEEAPAAAESEAPVEEDEEADSLAQAAAALGDTDEQDADAEYKARLRKFTRELKK QPGVWYIIQCYSGYENKVKANLDMRAQTLEVEDDIFEVVVPIEQVTEIRDGKRKLVKRKLL PGYVLVRMDMNDRVWSVVRDTPGVTSFVGDEGNATPVKHRDVAKFLMPQEQAVVTG EAAAAAAEGEQVVAMPTDTKKPQVAVDFTVGEAVTILTGAFASVSATISSIDPELQKLEVL VSIFGRETPVDLSFDQVEKVS |
| 6 | MINHEVRTHRSAEEFPYEEHLAHKIARVAADPVEVAADTQEMIINRIIDNASVQAASVLRR PVSSARAMAQVRPVTDGRGASVFGLPGRYAAEWAALANGTAVRELDFHDTFLAAEYSH PGDNIPPILAAAQQAGKGGKDLIRGIATGYEIQVNLVRGMCLHEHKIDHVAHLGPSAAAG IGTLLDLDVDHHLPGNWSGIAHHHGDEAVP |
| 7 | MSTAYFIAHGNDTYRPTEHSSGAWRDDELHLAPVAGLVIHHMERWRREVVGDALVFSR FSLEVLGQIARDDVTLRTEIVRPGRTIELIETVAEINGRVTIRARAWLLKTSDLAHISGDTFEAL PSMTELADVKSAFQWSGGFMDSIVFVEDAEKIPGKNRAWITGDVQLVDGEDSEPLAEFS KFLDVANGVAVREDPKTWMFPNVDLTFHLFRQPVGTKVGFDTRAAFGPNGIGLTTSVIH DAEGPVGTLNQSLTVRPIAELNK |
| 8 | MSAFDSILNVEEWISDHYLTNDDAKGASFSKRVRERIKEWKTTEDATQQSGPLTRFSSNR LQLQHALSELDDATTAASLVASALGYGVPSARHAQRGSDTISYSSWVGNAGSVEFLAATP AESFEENFRSLPLEPVAVNDKPQDITAAKLVGQIFLSDTPPAFVVITAGKWVVLAERETWPL GRHLAIDISLVVERNDTKAQGEMQQTVVALARENTERAADGTTWWEETIEQSREHAVKV SGELRSAVRESIEILGNDVLTRYEAKELSTAEIDGGELAKQSLRYLYRILFLLFAEASPELEILPT GTPEYDEGYGLARLRELILNPPVTQHARTGTHLYDSLQLLFTLVDKGHHPTDAKAVAFDAE AGEEGLHFRNLSADLFLPAATELIDRVGLSNEALNKVLENLLLSRVQSGKDRGFISYATLGV TELGQVYEGLMSYTGFIAQEDLFEVAPHGKADKGSWMLPVSKADEVPADSFIEVDQEAP GGGVIKVRKRHPRGSFVFRQSSRDRERSASFYTPQVLTSFTVTQAIEELQASKRITTANDVL SLTICEPAMGSGAFAVEAVRQLAELYLELRQEELEQQIPAEDRAKELQKVKAHIALHQVYG VDLNSTAVELAEISLWLDTMNAEMDAPWYGLHLRNGNSLVGATRSLYAPSLLNKKAWLT ATPTRYRLDDIAQAIDENKAEPLFNHGIHHFLLPSTGWGATADAKDLKDLMATEIKELKS WRTSIRASLSKTQIKQLNNLALRVETLWRFVLMRIRIAESQISRSTTLWGQEPAEVSEVVTR EQIEQDLFGNIDGAYNRLRLVMDAWCALWFWPLDAVATAEHPERPALPDLDEWLATLT EILGIDLPLKSKNENQIVLGPDTNWLAINDAEATDLGFSGALSFERVSANHPWINVARQV AKQQSFFHWDLDFAHVFAKGGFDLQVGNPPWVRPDVNFEDLLAEHDPWWAVMSKPT QASKKERQKNFHNNPKSLEHVVSGAGETVATSAILGSVTLYPHLKDQRPDLYRGFMEKT WSNASPAGAISLIHPESHFTEKKAAPLRRGAYERLRRHWQFINELILFDVHDLVKYGVHVY GAPQESINFLSAASLYHPQTVLDSFDHDGSGNLPGLKDDNGNWDRRPHKDRIQLVNAD TLTVWKSILEDEQTPYLDTRMVYTVNTEAAAVLEKLASAPRIKELGLQFSSGWNETTDKKK GYFDVGWGYPASWSDAILQGPHLGVATPMIKQPNPTMKHNQDWSEIDFEAIPANFIPA TAYQPDRQTKPTYDADYGTWTFGDKQVPVADTFRIAWREMAATTGFRTVYPSVIPPGAN HVHTVNSAASRSNLKTILVGAQLGAILSDYFARSSGSSHIFNDIVRKIPLPNFTSLEKQFART YLRLNCLTSAYAPLWEEITGEPWDVQVPLRNAEQRRAAQNDIDAMVALSLGISADELCMI YRTQFPVMRRYDQEDHFDANGRKVPKEIIKLQQKLKDGQELSVEKRTWVHPQSEVSYTFE YPFRVLDREADLRAAYAKFENQLKEP |
| 9 | MNSTILLAQDAVSEGVGNPILNISVFVVFIIVTMTVVLRVGKSTSESTDFYTGGASFSGTQN GLAIAGDYLSAASFLGIVGAISLNGYDGFLYSIGFFVARLVALLLVAEPLRNVGRFTMADVL SFRLRQKPVRVAAACGTLAVTLFYLIAQMAGAGSLVSVLLDIHEFKWQAVVVGIVGIVMIA YVLLGGMKGTTYVQMIKAVLLVGGVAIMTVLTFVKVSGGLTTLLNDAVEKHAASDYAATK GYDPTQILEPGLQYGATLTTQLDFISLALALCLGTAGLPHVLMRFYTVPTAKEARKSVTWAI VLIGAFYLMTLVLGYGAAALVGPDRVIAAPGAANAAAPLLAFELGGSIFMALISAVAFATVL AVVAGLAITASAAVGHDIYNAVIRNGQSTEAEQVRVSRITVVVIGLISIVLGILAMTQNVAF LVALAFAVAASANLPTILYSLYWKKFNTTGAVAAIYTGLISALLLIFLSPAVSGNDSAMVPG ADWAIFPLKNPGLVSIPLAFIAGWIGTLVGKPDNMDDLAAEMEVRSLTGVGVEKAVDH |
| 10 | MTTALEEQNAQQAATAGRVVRVIGAVVDVEFPRGELPALYNALTVEVTLESVKKTVVLEV AQHLGDNLIRTIAMAPTDGLVRGAAVTDTARPISVPVGDVVKGHVFNALGDCLDDVSLN NNPEIERWGIHREPPSFDQLEGKTEILETGIKVIDLLTPYVKGGKIGLFGGAGVGKTVLIQEM ITRIAREFSGTSVFAGVGERTREGTDLFLEMEEMGVLQDTALVFGQMDEPPGVRMRVALS GLTMAEYFRDVQNQDVLLFIDNIFRFTQAGSEVSTLLGRMPSAVGYQPTLADEMGVLQE RITSTKGRSITSLQAVYVPADDYTDPAPATTFAHLDATTELDRSIASKGIYPAVNPLTSTSRIL EPAIVGERHYEVSQRVIGILQKNKELQDIIAILGMDELSEEDKITVARARRIERFLGQNFFVAE KFTGLPGSYVPLTDTVDAFERICNGDFDHYPEQAFNGLGGLDDVEAAYKKLTGK |
| 11 | MTSPVENSTSTEKLTLAEKVWRDHVVSKGENGEPDLLYIDLQLLHEVTSPQAFDGLRMTG RKLRHPELHLATEDHNVPTEGIKTGSLLEINDKISRLQVSTLRDNCEEFGVRLHPMGDVRQ GIVHTVGPQLGATQPGMTIVCGDSHTSTHGAFGSMAFGIGTSEVEHVMATQTLPLKPFK TMAIEVTGELQPGVSSKDLILAIIAKIGTGGGQGYVLEYRGEAIRKMSMDARMTMCNMSI EAGARAGMIAPDQTTFDYVEGREMAPKGADWDEAVAYWKTLPTDEGATFDKVVEIDGS ALTPFITWGTNPGQGLPLGESVPSPEDFTNDNDKAAAEKALQYMDLVPGTPLRDIKIDTV FLGSCTNARIEDLQIAADILKGHKIADGMRMMVVPSSTWIKQEAEALGLDKIFTDAGAEW RTAGCSMCLGMNPDQLKPGERSASTSNRNFEGRQGPGDRTHLVSPAVAAATAIRGTLSS PADI |
| 12 | MSFSAEKGTHLSEFIADLGFDLDEFQIKGCHAVEEDHGVLVCAPTGAGKTIVGEFAVSLALS RGTKCFYTTPIKALSNQKYHDLVAKHGSDAVGLLTGDVSINHDADIVVMTTEVLRNMIYA GSFALERLSHVVMDEIHFLADASRGAVWEEVILNLDDSVNIIGLSATVSNSEEFGEWLTTV RGDTRVIVTDHRPVPLDQYMMVQRKVMPLFEPGTDGRVNKELEATIDRLNSKQSEQGR AAYRSGEGFRARSKGDKQDSRTGKPREQDRHRPLGRPEVLSILKGINMLPAITFIFSRAGC DGALYQCLRSKLVLTDQAESEEIARIVDAGVVGIPEEDLQVLNFKQWRAALMRGFAAHH AGMLPAFRHIVEELFVKGLVRAVFATETLALGINMPARTVVLEKMVKFDGEGHVDLTPGQ YTQLTGRAGRRGIDVLGNAVVQWSPALDPRWVAGLASTRTYPLISTFQPGYNMSVNLLK TIGYEPSLRLLEKSFAQFQADGSVVGDVREIERAEAKVAELRAQLNKEIAATNPAVREDQD AVEVFIDYMELRRELNEEEKLNRKQSIEDRNVETVRVLSKLQIGDVIAMPGRKRPVLAVVM TPANQSRDPRPWVTTESGWSGRVDAESFTNPPITIGHMRLPRQAIEEPRRNARRVQELFR REHFKRPNKMREFARVRPNEAVTKLRNAIRDHEAYHWPDREHLARTAERMIRKERDLAK LTGNVDKARETLGRTFERILSLLSEMDYVDYSNPDNPVITDEGERLAKIHSEADLLVAQCLK RGIWDNLDPAELAGVVSMCTFENRRETGGEAQAVTEAMADSMNSVERIWGELVEDERR HRLPITRQPEAGFATAIHQWASGAPLGYCMAAAAENGAELTPGDFVRWCRQVIDLLEQV AKTAYFDETTRNARQAIDAIRRGVVAIGS |
| 13 | MTTNAPDGATNNINNAHSGAVGKPKVQLSDAEIQKYTAAFAGTTTTKPWELEVTTKFLK KIAWVAVVVIMAVHIFMGAVVDVDFTGAAVTFVDTLAFPALGIIFSVLVFLGLTRPRVRAN EDGVEVRNFIGTRFYPWVVIYGMSFPKGSSVARLELPDFEFVPMWAFQSRDGEDVVRAV ATFRDLENKYMPED |
| 14 | MKLFSRTSLVALGTAAAITLSGVTAPAFADEDSNAAVSALKTAEDNTPEAPGASTPLKLEQ PGTITGVPGKAITPVTVKVIAGEAESFTSDNLPSGLLIDNTGKITGTPKKEFTGSAKIIAKNEA GVEAEVYYVNFDFNEEPSSEEPSSGSSDTDNIENWIKIITAVIGALTTILTFSTKLDSFLK |
| 15 | MVSQTERQHAIASLLAPTGAVSVGDLAEHFHVTTETVRRDLRIMESLGLLQRVHGGAISPE PMGTSPPRLKPALVKGMPPEPRVLELAETAVSLITPLARSIFLDSGLACTAIATVLGDPPEDA RWTVVTSSPGAVIALSATDATSTVVLHGQVHGNCSSIIGSTAVDMISQLRADIAFVEVDAI QSDTSLCTFFPETIPIKQAMIKNAAFTVAVLSPRSPQDQELQLLKHPFSTLADFDALVTDDH TLDFPVLPDHNFQVVTP |
| 16 | MTQQLNHAKVNQHPGQATLPETAEGQVRTYEVKTYGCQMNVHDSERLSGLLEEAGYV AAPEDTTPDLVVFNTCAVRENADMRLYGTLGNLRSVKEKNPGMQIAVGGCLAQKDKDT VVKKAPWVDVVFGTHNIGSLPTLLQRAEHNAQAEVEIVDSLEQFPSVLPAKRESAYAGW VSVSVGCNNTCTFCIVPSLRGKEQDRRPGDILAEVQALVDQGVTEVTLLGQNVNAYGVN FVDPELERDRSAFSKLLRACGEIEGLERVRFTSPHPAEFTSDVIDAMAETPNICPQLHMPLQ SGSDKVLKEMRRSYRSKKFLSILDEVRAKIPHASITTDIIVGFPGETEEDFQATLDVVKKARFT SAYTFQYSPRPGTPAAEYENQLPKEVVQERYERLMVVQEQVCEEENQKLIGTTVELLVQA GGGRKNDATKRMSGRARDGRLVHFAPEGDIDGEIRPGDFVTVTVTEAKPFFLIADSGVQT HRRTKAGDNSAVGQVPTTAPIGVGLGLPQIGAPKVAPATESACCSIN |
| 17 | MAVAKDVLSFSRPIPALHAIIGALGWFGMFAVSLWSTL |
| 18 | MSITTHVQALTTALNAIDNHLASMLDHGVTPDQYKAIEPDLIALEHTINHHATIAAQTTAL AERTNAAHTIGSTHLIDYLTTTFGLSKARAHHRINLAHSLYPIPKPNPGSGNGGNGGNPD GGPDGGDSGDDDSGDDDPDPEPDKPEDGKPDGDKPRGPRISAEKHAIITDELARLNPNT TPSAEELRTQALSQAIWRTPEDLRTWLRHHVTTANKNNPNPITAMKRRYLAVGKPDADN MVRISGLVPAATAALITANTAPLTKRGNLVDLPAAEDMRTRGQRHADALHHIMEIYNHGI VTPARGGTASIIISMTTDDLDEINHGDHGDGSLLNNLYPTNTGYSLNLAEIMNLIAAKYDF AVLLDGETGQPLNVNRMQRSANLTQRIALFASELVCSAPNCDRPQLECEVHHLDPWMR GGLTNLVNLTHQCFNHHPRNDDSRSGVNGKGFMDRDPATGRVGHYPVGGEGPVFNR SAAADRSGGAWARRKHHGPPPTPPDPPDPPGPEPDPGDEGRLF |
| 19 | MRMLFENSIVCHLLFLSLPQITLGDVWWVMPGGGSPNYRSL |
| 20 | MPKPLPPETRRKIIDFDPFAPNSPSIEEFCSRLKISRRSFYNIRNRYQQDANAALHPHSSAPI TARRTYDESITSTLLSIRARLKAQGWEYGPISIRFEGISTGELTAPIPSVSTIARLLRAAGAVES NPKKRPKSSVVRFQRGQAMEMWQIDGFIYTLHDTDLTRVTIYQILDDATRFDVGTCVFPA NENSVDARTALEQAIAHFGAPHELLSDNGSAFNRMRQGYVGSLESYLATVGCLSITGKPG HPQTQGKNERSHRTLFRFLQAHQPHTLEECAHYIEQFRDHYNNRRPHQGLPNNLSPAA AWEIVGCVEQQPPIDPVVLQQQADHYARRRIEKQSDVPLVRQEESVPVSKMQQTEQRR RVRRFGKKPVDPDVFTFAGNNQKVVFQGMRISVPRTMRDREFYRTVTATELGFWDAITG ELELSIPLPVVAIARGKSYINSYNIRGVWMQHPTPLWQRKRDEAEKRFKSIDPGDLLR |
| 21 | MTTAAIRGLQGEAPTKNKELLNWIADAVELFQPEAVVFVDGSQAEWDRMAEDLVEAGTL IKLNEEKRPNSYLARSNPSDVARVESRTFICSEKEEDAGPTNNWAPPQAMKDEMSKHYA GSMKGRTMYVVPFCMGPISDPDPKLGVQLTDSEYVVMSMRIMTRMGIEALDKIGANGS FVRCLHSVGAPLEPGQEDVAWPCNDTKYITQFPETKEIWSYGSGYGGNAILAKKCYALRIA SVMAREEGWMAEHMLILKLINPEGKAYHIAAAFPSACGKTNLAMITPTIPGWTAQVVGD DIAWLKLREDGLYAVNPENGFFGVAPGTNYASNPIAMKTMEPGNTLFTNVALTDDGDI WWEGMDGDAPAHLIDWMGNDWTSESDENAAHPNSRYCVAIDQSPAAAPEFNDWEG VKIDAILFGGRRADTVPLVTQTYDWEHGTMVGALLASGQTAASAEAKVGTLRHDPMAM LPFIGYNAGEYLQNWIDMGNKGGDKMPSIFLVNWFRRGEDGRFLWPGFGDNSRVLKW VIDRIEGHVGADETVVGHTAKAEDLDLDGLDTPIEDVKEALTAPAEQWANDVEDNAEYLT FLGPRVPAEVHSQFDALKARISAAHA |
| 22 | MTTAVDQNSPPKQQLNKRVLLGSLSGSVIEWFDFLVYGTVAALVFNKMYFPSGNEFLSTI LAYASFSLTFFFRPIGGVIFAHIGDRIGRKKTLFITLMLMGGGTVAIGLLPDYNAIGIWAPILL MFLRILQGIGIGGEWGGALLLAYEYAPKKQRGLYGAVPQMGISLGMLLAAGVISLLTLMPE DQFLTWGWRIPFVGSILLVFIGLFIRNGLDETPECKRIRDSGQQVKMPLKEVLTKYWPAVL VSIGAKAAETGPFYIFGTYIVAYATNFLNIRDNIVLLAVACAALVATIWMPLFGSFSDRVNR AVLYRICASATIVLIVPYYLVLNTGEIWALFITTVIGFGILWGSVNAILGTVIAENFAPEVRYTG ATLGYQVGAALFGGTAPIIAAWLFEISGGQWWPIAVYVAACCLLSVIASFFIQRVAHQEN |
| 23 | ATGACAAAAATCAAGAGTGGGGAGGCGTCGACAAGCATTGTTGAGCGCGCCTTAAA GCGCCCCGAACTGACCAGCCTGCTTGGCGCCGTGCTTGTTTTTACGCTGTTTATGGTG GTCGCGCCGGCATTTAGGTCATGGGATTCGATGGCGACCGTGCTGTATGCGAGTTCC ACGATCGGCATCATGGCGGTTGCCGTGGGCCTGCTGATGATCGCTGATGAATTCGAC CTGTCCACCGGCGTTGCCGTGACAACTGCAGCGCTGGCGGCCTCGATGTTTAGCTAT AACCTGTGGCTGAACACCTGGGTGGGCGCGCTGATTGCATTGGTGATTTCGCTGGCC ATCGGCTTTTTCAACGGCTTTTTGGTAGTGAAAACCAAGATTGCATCCTTCCTGATCAC CCTTGCCACTTTCCTTATGCTGCAGGGTATTAATCTGGCGGTCACCAAGCTGATTTCCG GCACCGTGGCCACGCCAACCATCGCGGATATGGAAGGTTTTCCTTCAGCGCGTGCGG TGTTTGCCAGCTCGATTCCCATCTTTGGTGTGATTATTCGCATCACTGTTTTTTGGTGGC TGCTGTTTGTTATCGTCGGCACTTTTGTGTTGTTTAAGACGCGCATCGGCAACTGGATT TTTGCGGTCGGTGGCGATGAAGAGGCAGCTCGCGCAGTCGGCGTTCCCGTGCGTGG CGTGAAAATCGGCCTGTTCATGTTCGTTGGTTTTGCCGCCTGGTTTGTGGGCATGCAC AACCTGTTCCTCTTTGATTCGATTCAGGCTGGTCAAGGCGTGGGTAATGAGTTCCTCT ACATCATCGCTGCGGTGATCGGAGGCATCTCCATGACTGGTGGCCGCGGAACAGTG GTGGGCACAATGATTGGTGCACTCATCTTTGGAATGACCAACCAAGGCATTGTTTATG CAGGTTGGAACCCTGACTGGTTCATGTTCTTCCTCGGCGGCACCCTACTTCTGGCTGT TTTGCTCAATCACCGATTCGAGCGTTTCAACAAGGAGCGATCATGA |
| 24 | ATGCACCACGAGCAACCCGAAGGGTGCGAAGTGGGCATTCGTAGAACAATCCCAGA GGAAAGCCGTACGGCTTTCCTCGACATGATCAATCAAGGTATGTCAGGTCTTGCTGC GTCTACAGCGGTCGGGGTCAGTGAATTCACCGGGCGAAAGTGGGCGAAGGCCGCCG GGGTGAAACTGACCCGCGGCCCGCGAGGTGGCAATGCTTTTGACACCGCCGAGAAA CTTGAGATTGCAGCCAGCATGCTAGAGAAAGGATGCCTACCCCGAGAAATCGGCGA GTATGTCGGCATGACTCGGGCCAATATATCCCTATGGCGCAAACAAGGCCCAGACAA GCTTCGCCAACGCGCAGCCACCTTGCGCACCGGCAAGCGAGCAGCTGAATTCATCCA CGCCCCGGTGATGGGCCCTTATTATGGGCCACGCACACTCCATCAAGTGTTGCGTGA GGACTACACAACACTGTTTGACGAGTTATCTGCGTTGGGGTTGCCAGCACAGGTGTG TGGGGCCTTACTTCATCTTGCTCCACCACCATCATTACGCTTTTCTTATATGTCGTGTGT AGTGCCGTTATTTGCTGATGAAATCAAAGTCGTAGGACAAGGCACACGATTATCGTTA GAAGAGAAAATGATGATCCAACGTTTCCATGACACCGGGGTCAGTGCAGCAGAAATC GGTCGACGCCTGGGTCGGTGTCGGCAAACAATTTCCAGGGAACTTCGACGTGGTCAA GATGATGATGGACGTTATCGTGCACGCGACTCCTATGAAGGTGCGATCAGGAAACTA GCGCGTCCGAAAACACCGAAACTTGATGCCAATCGTAGGCTTCGGGCTGTGGTGGTC GAGGCGTTGAATAATAAATTATCTCCGGAGCAGATTTCTGGTCTTTTAGCCACCGAGC ATGCTAACGATAGCTCTATGCAGATTAGTCATGAAACTATTTACCAGGCGTTATATGTT CAAGGTAAAGGGGCGTTGCGTGATGAATTGAAGGTGGAGAAATTTCTTCGTACCGGT CGGAAGGGACGTAAACCGCAGTCGAAGTTGCCATCGAGAGGTAAGCCGTGGGTGGA GGGTGCGTTGATTAGTCAACGCCCAGCAGAAGTTGCTGATCGTGCTGTGCCTGGGCA CTGGGAGGGCGATTTAGTAATTGGTGGTGAAAACCAAGCGACAGCGTTGGTGACGTT GGTGGAGCGCACGAGCCGGTTGACGTTGATTAAGCGGTTGGGGGTTAATCATGGGG CGTCGACTGTGACGGATGCGTTGGTGGAGATGATGGGTGATTTGCCGCAGGCGTTGC GTCGGAGTTTGACGTGGGATCAGGGTGTGGAGATGGCAGAGCATGCGCGGTTTAGC GTGGTGACCAAGTGTCCGGTGTTTTTCTGTGATCCTCATTCGCCGTGGCAGCGTGGGT CGAATGAGAATACGAATGGATTGGTCAGGGATTTTTTCCCGAAGGGCACTAATTTTGC TAAAGTAAGTGACGAAGAAGTTCAGCGGGCACAGGATCTGCTGAATTACCGGCCGC GGAAAATGCATGGTTTTAAAAGCGCGACGCAGGTATATGAAAAAATCGTAGTTGGTG CATCCACCGATTGA |
| 25 | ATGGGTTCCATTACTCCGCAGAAGCGGCCTCGCGTGGGGTCTCACATCGCGAACAAG GGTCAAGAGACTGATATCGGGCGAAAACGCCGAGCTCGACGCATCAATCGCACACT CACCGTGGCATATCCGGATGCGCACTGCGAATTAGATTTCACCAATCCGCTAGAACT CACGGTCGCCACCATTTTGTCCGCCCAGTGCACGGACGTTCGCGTGAACCAGGTGAC GCCCGCGTTGTTCAAGCGCTATCCGACGGTCACAGATTACGCCAACGCCGATCGCAC GGAATTGGAGGAGTTCATCCGTCCGACAGGCTTTTACCGCAACAAGGCCACTTCTTTA ATCGGCCTGGGTGAGGCACTAATTTCGCTTCACGACGGCCAGGTCCCCGGTACCCTT GAGCAGCTAGTTGAGCTGCCGGGGGTCGGGCGGAAAACCGCCAACGTGGTGCTGGG AAATGCTTTCGGTGTTCCGGGAATCACGGTGGATACACACTTTGGCAGGTTGGTGCGT CGCCTGAAGCTCACTGATGAAGAAGATCCCGTCAAGGTGGAAAAAGTGATGAACGA ACTCATCGAAAAGCCTGAGTGGACCATGTTTTCACATAGGCTGATCTTCCACGGACGT AGGATATGTCATAGTCGACGCGCCGCCTGTGGAGCCTGCATGCTGGCAGCTGATTGC CCATCCTTTGGTTTGGAGGGGCCGTCAGATCCATTTGAGGCGCAAAAACTCATTAAAA GTGATGATAGGGAGCACCTGCTGAAAATGGCAGGAATGTAG |
| 26 | ATGGAATTAAGGGAATACGCGACAATCCTGATGAAGAATTGGGTGTTGATCGTTATC GCATCCATTCTTGGAATTGCAGCGGGCGCTGGTTTTTCCCTTCTAGCTACACCGGAGT ATCAGTCACGTACTCAGTTGTATGTATCGGTGCGGTCGGGGGCTGGGACAACCTCTG ACATGGTTCAGGGTGCTAACTTTTCGCGTCAGATTGTGAATAGTTATGTTGATGTCATT AAGACGGGTGTTGTTCTAGAGCCGGTTGTTGATGAGCTTGGTCTGGAGTTGACGGCT AACCAGTTGAGTTCTCATATCAGTGCGGCTTCTCCTGCTGATACTGCGTTGATCAACAT CACTGCTTCTAGTCCTTCTCCTCAGCAGGCGGCTGAGATCGCCAATGCAGTGGGGGA GAGTTTCAAGAATGTGATTCAGACTGAATTGGAACCAGACTCCGGCGATGGCATGAG CCCGATTAACCTGACTACTACTCAGGTGGCTCTGGAACCATCTTCTCCGGTCAGTCCT AATGTGATGATGAATATCCTCCTCGGCCTACTTGTAGGTCTTGCAATAGGTGTTGGTAT TGCAGTGCTTCGCGCGGCTTTGGATACTCGTATTCATTCCTTGCGCGATATTGAAGAG GTTACTGATAAGCCACTTTTGGGCGGAATCATTGCGGATTCTGAAGTTGAGAAGCACC CGTTGATCATTAAGCACAAGCCGCATAGTCCTATTGCGGAGTCCTTTCGTGCGTTACG TACTAACCTGCAGTTCCTTAACGTCGGCGGCTCATCTTCAGTATTTGTTATCTCCTCTG CTAATCCTGGTGAGGGTAAGTCAACCACTTCTGTAAACCTAGCTTTGGCGCTTGCAGA GGCCGGCTCCCGTGTAGCGCTGATCGAAGCTGATCTTCGCTTGCCACGAGTGAGCAA GTACCTCGGAGTTGAGGGCAACGCAGGTCTGACTGACATTCTCATTGGCAAGGCCGA GGTTAATGATGTGTTGCAGCGTTGGGGTAGGACTCAGTTGTACTATCTCCCGGCAGG GCGCATTCCGCCGAACCCGAGTGAGTTGCTTGGTTCAGCTGAGATGGACAAGGTCAT TGCGGAGCTTGAGGAAAGCTTTGATTATGTGATCATCGATGCCCCTCCGGCGTTGGC GGTTACCGATGCTGCAGTTATTGGTCATGGCAAGGCTGGCATCCTGATTGCGGTCTCC GCAGGTTCTACAAAGAAGCCTGAGTTGGAAGCTACGCTGTCCACGCTTGAGAATGCG GATGCCAATGTTGTTGGCGTTGTCGCTACGATGCTCCCGCCTAAGTCTGTGGCTGGTT ATGGCTACGGAAATTACGGCTACGGCGACACCTCCAAAATCAATGCCCCTAAGCCCG ACAACACCGAACTAACCACCACCGATGCTTCCAAGGCCAACAATGAGCAATAG |
| 27 | ATGAGCGATGAGAACATTAACGAGTTTGAGCAGGACGAGGATCTGAACTTCGGCGC GAGCTTTAGTGATGAATTCGCAGATGACGATTTCGATGCAGAAGCAGACGTAGAAGC AGATGCTGCTGCAGAGGCCTCTGCCCTGGAAGCTGAGCAGGATCTGGAAGAAGAGA CCCTAGATGCTCCAGAAGAAGCCGCAGAAGAAGCTCCTGCTGCTGCAGAGTCCGAA GCTCCAGTAGAAGAGGACGAAGAGGCTGACAGCCTTGCTCAGGCGGCTGCTGCACT TGGTGACACCGATGAGCAGGACGCGGATGCAGAGTACAAGGCTCGTCTGCGTAAGT TCACTCGTGAGCTGAAGAAGCAGCCTGGTGTTTGGTACATCATTCAGTGCTACTCCGG CTACGAGAACAAGGTGAAGGCGAACCTTGACATGCGTGCTCAGACCCTTGAGGTTGA GGATGACATCTTTGAGGTTGTTGTTCCTATCGAGCAGGTCACTGAGATCCGTGATGGT AAGCGCAAGCTGGTTAAGCGTAAGTTGCTGCCGGGCTACGTTTTGGTCCGCATGGAC ATGAATGACCGCGTGTGGTCTGTTGTTCGCGATACACCTGGTGTGACCAGCTTTGTGG GTGACGAGGGCAATGCAACTCCTGTGAAGCACCGCGATGTTGCGAAGTTCTTGATGC CTCAGGAGCAGGCTGTTGTCACCGGTGAGGCTGCTGCTGCGGCTGCCGAGGGTGAG CAGGTTGTGGCTATGCCTACCGATACCAAGAAGCCTCAGGTTGCTGTGGACTTCACT GTTGGTGAGGCTGTGACCATTCTGACTGGTGCTTTCGCTTCTGTTTCTGCAACGATTTC TTCTATCGATCCTGAGCTGCAGAAGCTGGAAGTTTTGGTGTCCATCTTTGGTCGTGAA ACTCCTGTTGATCTCAGCTTCGACCAGGTTGAGAAGGTTAGCTAG |
| 28 | ATGATTAACCACGAAGTGCGCACCCACCGTTCTGCGGAAGAGTTCCCGTACGAGGAG CACCTGGCTCACAAGATCGCTCGCGTTGCAGCCGACCCAGTTGAGGTTGCTGCGGAC ACTCAGGAAATGATCATCAACCGCATCATCGACAATGCATCGGTGCAGGCAGCTTCC GTGTTGCGTCGACCAGTTAGCTCTGCCCGTGCGATGGCACAGGTCAGGCCAGTTACC GATGGTCGGGGTGCATCTGTTTTCGGTCTGCCAGGACGTTATGCCGCGGAATGGGCT GCGCTTGCTAACGGCACTGCGGTGCGTGAGCTTGATTTCCATGACACGTTCCTCGCTG CGGAATACTCCCACCCAGGAGATAACATTCCTCCGATTTTGGCTGCAGCACAGCAGG CTGGAAAAGGTGGCAAGGATCTGATCCGTGGCATCGCTACTGGGTATGAGATTCAGG TTAACTTGGTGCGTGGAATGTGCCTGCATGAGCACAAGATTGATCACGTTGCTCATCT TGGACCATCAGCGGCTGCTGGTATCGGAACCTTGCTAGACCTAGATGTGGACCACCA TCTACCAGGCAATTGGTCAGGCATTGCACACCACCACGGCGACGAGGCAGTCCCGTA A |
| 29 | ATGTCAACTGCATATTTTATTGCGCACGGAAACGATACGTACCGCCCAACGGAGCATT CCAGCGGTGCGTGGCGCGATGATGAACTCCACCTTGCACCGGTTGCCGGTTTGGTGA TTCACCATATGGAACGTTGGCGCAGGGAAGTGGTTGGCGATGCTTTGGTGTTCAGCC GTTTTAGTTTGGAGGTCTTGGGCCAGATCGCTCGGGATGATGTCACCTTGAGAACAG AGATCGTTCGTCCCGGGCGCACCATTGAACTTATTGAAACCGTGGCTGAAATCAACG GCAGAGTGACGATTCGTGCACGCGCCTGGTTGCTGAAGACCTCTGATCTTGCCCATAT TTCTGGCGATACTTTTGAAGCGTTGCCGTCAATGACTGAACTGGCAGATGTTAAGTCA GCGTTCCAGTGGTCTGGTGGATTTATGGACAGCATTGTGTTTGTTGAGGACGCTGAGA AAATTCCGGGCAAAAACCGCGCGTGGATTACCGGCGATGTGCAGTTGGTTGACGGC GAAGATTCTGAGCCTTTGGCAGAGTTTTCCAAATTCTTGGATGTGGCCAATGGCGTGG CGGTTCGGGAAGATCCAAAGACTTGGATGTTCCCGAACGTGGATCTTACTTTCCATCT GTTCCGCCAGCCGGTGGGCACCAAGGTGGGTTTTGATACCCGCGCGGCGTTTGGACC CAACGGCATCGGTTTGACCACGTCGGTGATTCACGATGCCGAAGGTCCTGTGGGCAC GCTTAATCAATCATTGACAGTGCGCCCAATTGCTGAGTTAAACAAGTGA |
| 30 | ATGAGTGCATTTGATTCGATCCTAAATGTTGAAGAGTGGATCAGTGACCACTACCTCA CCAATGACGATGCCAAAGGTGCCTCATTTTCCAAGCGGGTGCGCGAGCGCATTAAAG AATGGAAAACCACCGAGGACGCAACCCAGCAGAGTGGCCCTTTAACTCGTTTTTCCA GCAACCGCCTGCAGTTGCAGCATGCTCTTTCTGAGCTTGACGACGCCACCACCGCCG CCAGTTTAGTGGCCTCTGCACTGGGGTATGGTGTCCCCAGCGCGCGCCACGCGCAGC GCGGCTCCGACACAATATCCTATTCCTCTTGGGTGGGAAATGCCGGCAGTGTGGAAT TTCTTGCAGCGACTCCCGCTGAAAGCTTTGAAGAGAACTTCCGATCCCTTCCCCTTGA GCCAGTAGCGGTCAATGACAAGCCCCAGGATATCACCGCAGCCAAATTGGTGGGCC AGATTTTCCTTAGTGATACTCCCCCTGCTTTTGTTGTTATCACCGCTGGTAAATGGGTG GTTTTAGCCGAGCGTGAAACCTGGCCTCTAGGCCGCCACCTAGCTATTGATATTTCCC TGGTGGTGGAACGTAATGACACCAAAGCCCAGGGTGAGATGCAGCAGACGGTCGTA GCACTAGCCCGCGAAAATACCGAGCGTGCCGCCGATGGCACCACCTGGTGGGAAGA AACCATTGAGCAATCCCGCGAACATGCTGTCAAGGTTTCTGGCGAGCTACGCAGTGC GGTGCGTGAATCCATTGAAATCCTGGGCAATGACGTGCTCACACGCTATGAAGCTAA AGAGCTCTCCACCGCTGAGATCGACGGTGGCGAGCTAGCTAAGCAATCTTTGCGCTA TCTCTACCGCATTTTGTTCCTGCTTTTTGCCGAGGCTTCACCAGAGCTTGAAATCCTGC CAACCGGCACCCCGGAATATGACGAGGGTTATGGCTTGGCACGCCTGCGCGAGCTT ATTCTTAATCCCCCAGTTACCCAGCATGCTCGCACGGGCACCCACCTTTATGATTCCC TGCAGCTGCTGTTCACTCTGGTGGATAAAGGCCACCACCCAACAGATGCTAAGGCTG TAGCTTTTGATGCCGAGGCTGGAGAAGAAGGCCTGCACTTCCGCAACCTTTCAGCGG ATCTCTTCCTCCCTGCAGCCACAGAACTTATTGATCGAGTTGGTCTTTCCAATGAAGCC CTAAACAAGGTCTTGGAAAACCTCCTGCTCTCCCGGGTGCAATCCGGTAAAGACCGC GGCTTTATCTCCTATGCCACCTTGGGTGTTACCGAGCTTGGCCAAGTTTATGAGGGTC |
| | TGATGTCCTATACCGGCTTTATCGCCCAGGAAGATCTTTTTGAGGTTGCACCACATGG CAAAGCCGATAAAGGTTCCTGGATGCTCCCGGTCTCAAAGGCTGATGAAGTCCCTGC CGATAGCTTTATCGAAGTTGATCAAGAAGCCCCTGGTGGCGGCGTAATCAAGGTGCG TAAACGCCACCCGCGCGGATCATTTGTGTTCCGTCAGTCCTCTCGTGACCGCGAACG CTCAGCGTCCTTCTACACCCCACAAGTACTCACCAGCTTTACTGTCACCCAGGCTATT GAAGAACTCCAGGCATCAAAGCGCATCACCACAGCCAATGATGTTCTCAGCCTCACC ATCTGTGAACCTGCCATGGGTTCCGGCGCCTTCGCTGTGGAAGCAGTACGCCAATTA GCAGAGCTTTATTTGGAATTGCGCCAAGAAGAACTAGAGCAGCAGATTCCAGCGGAA GACCGTGCCAAGGAACTCCAAAAGGTCAAAGCGCACATTGCGCTGCACCAGGTTTAT GGTGTGGACCTTAACAGCACTGCTGTGGAGTTGGCGGAAATCTCGCTGTGGCTAGAC ACCATGAATGCAGAAATGGACGCACCTTGGTATGGCCTGCACCTGCGTAATGGTAAC TCCCTCGTTGGTGCCACCCGTTCGCTGTATGCACCTAGTCTGCTTAATAAAAAAGCCT GGTTAACTGCTACTCCAACCCGCTATCGGCTTGATGATATCGCGCAGGCTATTGATGA AAACAAAGCAGAACCCCTCTTCAACCACGGCATCCACCACTTCCTCTTGCCCTCTACT GGCTGGGGAGCCACTGCAGATGCCAAAGATCTTAAAGATCTTATGGCTACTGAAATC AAGGAGCTTAAATCTTGGCGTACTTCCATCCGTGCGTCTTTGAGTAAAACTCAGATTA AGCAGCTCAATAACCTTGCCCTACGCGTGGAAACACTATGGCGATTTGTGCTGATGC GTATTCGCATTGCAGAATCCCAGATCTCACGTAGCACTACTCTCTGGGGTCAAGAGCC AGCTGAGGTTTCGGAGGTTGTCACACGTGAGCAAATTGAACAAGACCTCTTTGGCAA TATTGATGGTGCATATAACCGTCTACGCTTGGTGATGGATGCTTGGTGTGCGCTGTGG TTCTGGCCTTTGGATGCTGTTGCTACCGCTGAGCATCCGGAGCGTCCAGCCCTTCCAG ATCTTGATGAGTGGCTAGCCACCCTGACGGAGATTCTGGGTATTGATCTCCCTCTGAA GTCCAAAAACGAAAATCAGATTGTCTTAGGTCCAGATACCAATTGGCTAGCCATTAAT GATGCCGAGGCTACTGATCTTGGTTTTTCTGGGGCATTGAGCTTTGAGCGTGTTAGCG |
| | CGAATCACCCGTGGATCAATGTTGCCCGCCAAGTGGCTAAACAACAGAGCTTCTTCC ACTGGGATCTAGACTTCGCCCACGTTTTTGCCAAGGGTGGATTTGATCTGCAGGTTGG TAATCCACCATGGGTGCGACCAGATGTGAACTTTGAGGATCTGCTTGCTGAACATGAT CCGTGGTGGGCTGTAATGAGTAAACCAACCCAGGCATCCAAAAAAGAACGCCAGAA GAATTTTCACAACAATCCTAAGAGCCTCGAACATGTGGTCAGTGGTGCAGGTGAAAC CGTGGCTACTTCTGCGATCCTCGGTTCGGTTACTCTCTATCCGCATCTTAAAGATCAAC GCCCGGACCTCTACCGGGGCTTTATGGAAAAGACTTGGTCTAATGCCTCCCCGGCAG GTGCGATCTCACTGATCCACCCCGAATCCCACTTCACTGAGAAAAAAGCTGCCCCGC TACGTCGGGGAGCATATGAGCGTCTGCGCCGCCACTGGCAGTTCATTAATGAGTTGA TTCTTTTTGACGTCCACGACTTGGTTAAATATGGCGTACATGTCTATGGCGCTCCGCAG GAATCTATTAACTTTTTAAGTGCTGCGTCGCTTTATCACCCACAAACAGTGCTTGATTC ATTTGATCATGACGGTTCAGGTAATCTCCCTGGTCTTAAAGACGACAATGGCAACTGG GACCGTCGCCCACACAAGGACCGTATCCAACTGGTCAATGCCGATACTTTGACGGTG TGGAAGTCCATCCTGGAGGATGAACAAACGCCATACTTGGATACCCGCATGGTTTAT ACCGTCAACACGGAAGCAGCAGCAGTGTTGGAAAAGTTGGCTTCTGCACCTCGTATC AAAGAACTCGGGCTGCAGTTCTCCAGTGGCTGGAATGAAACCACCGATAAGAAAAA GGGATACTTTGACGTTGGTTGGGGCTACCCAGCTTCCTGGTCTGATGCCATTTTGCAG GGGCCGCACCTGGGTGTTGCTACACCAATGATCAAGCAGCCCAATCCGACAATGAA GCATAATCAAGATTGGTCTGAAATTGATTTCGAGGCCATTCCTGCAAACTTCATACCT GCAACGGCGTACCAGCCCGATCGCCAAACAAAGCCCACTTATGATGCTGACTACGGC ACCTGGACTTTCGGGGACAAGCAGGTACCAGTTGCAGACACTTTCCGAATTGCATGG AGGGAGATGGCTGCCACCACGGGATTTAGGACTGTCTACCCATCAGTAATTCCACCG GGAGCCAACCATGTGCACACAGTTAATAGCGCTGCATCACGTTCAAACTTAAAAACC ATTCTCGTTGGAGCACAGCTTGGTGCAATTCTAAGTGACTATTTTGCTCGGTCCTCGG |
| | GTTCAAGCCACATATTTAACGACATTGTTCGCAAGATTCCACTTCCAAATTTCACATCC TTGGAAAAGCAGTTCGCCCGCACATACCTCCGCCTCAACTGCCTGACCTCAGCTTATG CCCCATTGTGGGAAGAGATCACCGGTGAGCCGTGGGATGTTCAGGTGCCTTTGCGCA ATGCCGAGCAACGTCGAGCAGCGCAAAACGATATTGATGCCATGGTGGCATTGTCTT TGGGTATTAGTGCTGATGAGCTGTGCATGATTTATCGCACTCAATTCCCAGTGATGCG TAGATATGATCAAGAAGATCATTTTGATGCCAATGGCCGTAAAGTTCCTAAAGAGATC ATCAAGCTGCAGCAGAAACTTAAAGATGGCCAAGAGCTCAGCGTGGAAAAGCGCAC CTGGGTGCATCCCCAATCAGAAGTGTCCTATACCTTTGAATATCCTTTCCGGGTGTTG GATCGTGAAGCTGATCTGCGTGCTGCATATGCAAAATTTGAAAACCAGCTAAAGGAG CCATAG |
| 31 | ATGAATTCCACTATTCTCCTTGCACAAGACGCTGTTTCTGAGGGCGTCGGTAATCCGA TTCTTAACATCAGTGTCTTCGTCGTCTTCATTATTGTGACGATGACCGTGGTGCTTCGC GTGGGCAAGAGCACCAGCGAATCCACCGACTTCTACACCGGTGGTGCTTCCTTCTCC GGAACCCAGAACGGTCTGGCTATCGCAGGTGACTACCTGTCTGCAGCGTCCTTCCTC GGAATCGTTGGTGCAATTTCACTCAACGGTTACGACGGATTCCTTTACTCCATCGGCT TCTTCGTCGCAAGGCTTGTTGCACTGCTGCTCGTGGCAGAGCCACTTCGTAACGTGGG CCGCTTCACCATGGCTGACGTGCTGTCCTTCCGACTGCGTCAGAAACCAGTCCGCGT CGCTGCGGCCTGCGGTACCCTCGCGGTTACCCTCTTTTACTTGATCGCTCAGATGGCT GGTGCAGGTTCGCTTGTGTCCGTTCTGCTGGACATCCACGAGTTCAAGTGGCAGGCA GTTGTTGTCGGTATCGTTGGCATTGTCATGATCGCCTACGTTCTTCTTGGCGGTATGAA GGGCACCACATACGTTCAGATGATTAAGGCAGTTCTGCTGGTCGGTGGCGTTGCCAT TATGACCGTTCTGACCTTCGTCAAGGTGTCTGGTGGCCTGACCACCCTTTTAAATGAC GCTGTTGAGAAGCACGCCGCTTCAGATTACGCTGCCACCAAGGGGTACGATCCAACC CAGATCCTGGAGCCTGGTCTGCAGTACGGTGCAACTCTGACCACTCAGCTGGACTTC ATTTCCTTGGCTCTCGCTCTGTGTCTTGGAACCGCTGGTCTGCCACACGTTCTGATGCG CTTCTACACCGTTCCTACCGCCAAGGAAGCACGTAAGTCTGTGACCTGGGCTATCGTC CTCATTGGTGCGTTCTACCTGATGACCCTGGTCCTTGGTTACGGCGCTGCGGCACTGG TCGGTCCAGACCGCGTCATTGCCGCACCAGGTGCTGCTAATGCTGCTGCTCCTCTGCT GGCCTTCGAGCTTGGTGGTTCCATCTTCATGGCGCTGATTTCCGCAGTTGCGTTCGCT ACCGTTCTCGCCGTGGTCGCAGGTCTTGCAATTACCGCATCCGCTGCTGTTGGTCACG ACATCTACAACGCTGTTATCCGCAACGGTCAGTCCACCGAAGCGGAGCAGGTCCGAG TATCCCGCATCACCGTTGTCGTCATTGGCCTGATTTCCATTGTCCTGGGAATTCTTGCA ATGACCCAGAACGTTGCGTTCCTCGTGGCCCTGGCCTTCGCAGTTGCAGCATCCGCTA ACCTGCCAACCATCCTGTACTCCCTGTACTGGAAGAAGTTCAACACCACCGGCGCTG TGGCCGCTATCTACACCGGTCTCATCTCCGCGCTGCTGCTGATCTTCCTGTCCCCAGC AGTCTCCGGTAATGACAGCGCAATGGTTCCAGGTGCAGACTGGGCAATCTTCCCACT GAAGAACCCAGGCCTCGTCTCCATCCCACTGGCATTCATCGCTGGTTGGATCGGCAC TTTGGTTGGCAAGCCAGACAACATGGATGATCTTGCTGCCGAAATGGAAGTTCGTTCC CTCACCGGTGTCGGTGTTGAAAAGGCTGTTGATCACTAA |
| 32 | ATGACTACAGCTCTTGAAGAGCAGAACGCACAGCAGGCAGCCACTGCCGGCCGTGT CGTGCGTGTCATTGGTGCGGTCGTCGACGTGGAGTTTCCCCGCGGCGAGCTGCCAGC ACTGTACAACGCACTTACTGTAGAGGTAACCCTCGAATCAGTTAAGAAGACCGTTGTT CTCGAGGTTGCTCAGCACCTCGGCGACAACCTCATCCGCACCATCGCTATGGCACCA ACCGACGGACTTGTCCGCGGTGCTGCTGTAACCGATACTGCACGCCCAATTTCCGTA CCAGTGGGCGATGTTGTTAAGGGCCACGTATTCAACGCTTTGGGCGACTGCCTAGAC GACGTTTCCCTGAACAACAACCCAGAGATCGAGCGTTGGGGCATCCACCGCGAGCC ACCATCATTCGATCAGCTTGAGGGTAAGACCGAGATCCTGGAAACAGGCATCAAGGT TATCGACCTTCTCACCCCTTACGTTAAGGGTGGAAAGATCGGCCTCTTCGGTGGTGCA GGTGTGGGTAAGACCGTTCTTATCCAGGAAATGATCACCCGTATTGCACGTGAGTTCT CCGGTACTTCCGTGTTCGCAGGTGTTGGTGAGCGTACCCGTGAGGGCACCGACCTCT TCCTCGAAATGGAAGAAATGGGCGTTCTCCAGGACACCGCCCTGGTGTTCGGTCAGA TGGATGAGCCACCAGGAGTCCGTATGCGCGTGGCTCTGTCCGGCCTGACCATGGCG GAGTACTTCCGCGATGTTCAGAACCAGGACGTGCTGCTGTTCATCGACAACATCTTCC GTTTCACCCAGGCAGGTTCTGAGGTTTCCACCCTTCTGGGTCGTATGCCTTCCGCCGT GGGTTACCAGCCAACCCTGGCTGACGAGATGGGTGTTCTCCAGGAGCGCATTACCTC CACCAAGGGCCGTTCGATTACCTCTCTGCAGGCCGTTTACGTTCCTGCCGATGACTAC ACCGACCCGGCTCCAGCGACCACCTTCGCTCACTTGGATGCAACCACCGAGCTTGAC CGCTCCATTGCTTCCAAGGGTATTTACCCAGCAGTGAACCCACTGACCTCCACCTCTC GTATTCTCGAGCCAGCAATCGTTGGTGAGCGTCACTACGAGGTTTCTCAGCGTGTCAT CGGCATTCTGCAGAAGAACAAGGAACTTCAGGACATCATCGCCATCCTTGGTATGGA CGAGCTTTCTGAAGAGGACAAGATCACCGTTGCACGTGCGCGTCGCATCGAGCGCTT CCTGGGTCAGAACTTCTTCGTTGCAGAGAAGTTCACCGGTCTTCCTGGCTCCTACGTG CCACTGACCGACACCGTCGACGCTTTCGAGCGTATTTGCAACGGCGACTTCGACCAC TACCCAGAGCAGGCTTTCAACGGCCTCGGTGGTTTGGACGATGTCGAAGCTGCATAC AAGAAGCTGACCGGAAAGTAA |
| 33 | ATGACCAGCCCCGTGGAGAACAGCACCTCAACTGAGAAGCTGACCCTGGCAGAGAA GGTGTGGCGCGACCATGTCGTGTCCAAGGGAGAAAACGGCGAGCCCGACCTCCTCT ACATCGACCTGCAGCTGCTGCATGAAGTGACCTCACCACAGGCATTTGACGGCCTGC GCATGACCGGCCGTAAACTGCGCCACCCAGAACTGCACCTGGCCACCGAAGACCAC AACGTGCCAACCGAAGGCATCAAGACTGGCTCACTGCTGGAAATCAACGACAAGATT TCCCGCCTGCAGGTATCTACTCTGCGCGACAACTGTGAAGAATTCGGCGTGCGCCTG CACCCAATGGGTGATGTCCGACAGGGCATCGTGCACACCGTCGGCCCACAGCTCGG CGCAACCCAGCCAGGCATGACCATTGTGTGCGGTGACTCCCACACCTCCACCCACGG TGCTTTTGGCTCCATGGCATTCGGCATCGGTACCTCAGAGGTTGAGCACGTCATGGCT ACTCAAACCCTGCCACTGAAGCCTTTCAAGACCATGGCCATTGAAGTTACTGGTGAAC TGCAGCCAGGTGTTTCCTCCAAGGACCTGATTCTGGCGATTATCGCCAAGATCGGCA CCGGCGGCGGACAGGGCTACGTTCTGGAATACCGCGGCGAAGCAATCCGTAAGATG TCCATGGATGCACGCATGACCATGTGCAACATGTCCATCGAAGCTGGCGCACGTGCC GGCATGATCGCCCCAGACCAAACCACCTTCGACTACGTTGAAGGCCGCGAAATGGC ACCAAAGGGCGCCGACTGGGACGAAGCAGTTGCTTACTGGAAGACCCTGCCAACCG ACGAAGGCGCAACCTTTGACAAGGTCGTAGAAATCGATGGCTCCGCACTGACCCCAT TCATCACCTGGGGCACCAACCCAGGCCAGGGCCTGCCACTGGGCGAATCCGTACCA AGCCCAGAAGACTTCACCAACGACAACGACAAGGCAGCAGCCGAAAAGGCACTGCA GTACATGGACCTGGTACCAGGAACCCCACTGCGCGACATCAAGATCGACACCGTCTT CCTGGGATCCTGCACCAACGCCCGCATCGAAGACCTGCAGATCGCCGCTGACATCCT CAAGGGCCACAAAATCGCCGACGGCATGCGCATGATGGTCGTGCCTTCCTCCACCTG GATCAAGCAAGAGGCAGAAGCGCTCGGACTGGACAAAATCTTCACCGACGCTGGCG CTGAATGGCGTACCGCAGGCTGCTCCATGTGCCTGGGCATGAACCCAGACCAACTGA AGCCAGGCGAGCGCTCCGCATCCACCTCCAACCGAAACTTCGAAGGACGCCAAGGA CCAGGAGACCGCACCCACCTGGTATCCCCAGCAGTCGCAGCCGCCACCGCAATCCG CGGCACCCTGTCCTCACCTGCAGATATCTAA |
| 34 | ATGAGTTTTTCTGCCGAAAAGGGCACCCACCTTTCAGAGTTCATAGCAGACCTGGGCT TTGATCTGGACGAGTTCCAAATCAAAGGCTGCCACGCTGTGGAAGAAGACCACGGTG TTTTAGTATGTGCGCCCACCGGCGCGGGCAAAACAATTGTTGGTGAATTCGCAGTGTC CCTCGCATTATCGCGGGGGACAAAGTGTTTCTACACCACCCCCATCAAAGCGCTGAG CAACCAGAAGTACCACGATTTGGTGGCTAAACACGGCTCCGATGCCGTTGGTCTGCT |
| | CACCGGTGATGTTTCCATTAACCATGATGCTGACATCGTGGTCATGACCACCGAAGTG CTGCGCAACATGATTTACGCGGGCTCTTTTGCGCTTGAGCGCTTAAGCCACGTGGTCA TGGATGAGATCCACTTCCTTGCTGATGCCTCCCGTGGCGCGGTGTGGGAAGAAGTGA TCCTCAACTTGGATGATTCCGTCAACATCATCGGTCTATCTGCCACGGTGTCCAACTC AGAGGAGTTTGGTGAGTGGCTGACCACTGTTCGCGGCGATACCCGTGTGATTGTTAC TGATCACCGCCCCGTTCCGCTTGATCAGTACATGATGGTGCAGCGCAAAGTGATGCC ACTGTTTGAGCCTGGCACCGATGGACGCGTGAACAAGGAGTTAGAGGCAACGATTG ATCGCCTCAACAGCAAGCAAAGCGAACAAGGCCGTGCGGCATACCGCTCTGGTGAA GGCTTCCGTGCACGCAGCAAAGGCGATAAGCAGGATTCTCGCACTGGTAAGCCACG GGAACAAGACCGCCACAGGCCACTGGGTCGGCCTGAAGTGCTCAGCATCCTCAAGG GCATCAACATGCTGCCAGCGATTACGTTTATCTTCTCCCGCGCGGGCTGTGATGGTGC GCTGTACCAATGCTTGCGTTCTAAGTTGGTCTTGACGGATCAAGCAGAATCAGAAGA GATTGCACGCATTGTCGACGCCGGCGTGGTGGGGATCCCCGAGGAAGACCTTCAAG TACTGAACTTTAAGCAGTGGCGTGCTGCACTGATGCGCGGTTTCGCAGCCCACCACG CGGGTATGCTTCCAGCGTTTAGGCACATCGTGGAAGAGCTCTTTGTTAAAGGTCTTGT CCGCGCGGTGTTTGCCACGGAAACCCTGGCATTGGGAATCAACATGCCAGCGCGCA CCGTGGTGTTGGAAAAGATGGTCAAATTTGACGGCGAAGGCCACGTTGATCTCACCC CTGGCCAATACACGCAGCTGACCGGTCGTGCTGGTCGACGTGGCATCGATGTGTTGG GTAATGCTGTGGTGCAGTGGTCACCAGCACTTGATCCACGATGGGTGGCAGGTCTTG CCTCTACGCGTACCTACCCGCTGATCTCTACGTTCCAGCCGGGCTACAACATGTCGGT TAACCTGCTGAAAACCATTGGTTATGAGCCTTCGCTGCGCCTTTTGGAAAAATCTTTTG CACAGTTCCAAGCCGATGGTTCCGTCGTGGGCGATGTGCGTGAAATTGAACGTGCAG AAGCCAAGGTGGCAGAATTGCGTGCCCAGCTGAACAAAGAGATTGCTGCCACCAAC CCTGCGGTGCGTGAAGATCAAGACGCAGTGGAGGTGTTTATCGACTACATGGAGCTT |
| | CGCCGTGAGCTCAATGAGGAAGAAAAACTCAATCGCAAGCAAAGCATCGAAGATCG CAACGTAGAAACAGTCCGTGTGCTGTCCAAGCTGCAAATCGGTGATGTCATTGCCAT GCCTGGTAGAAAACGACCAGTTCTCGCAGTAGTGATGACGCCTGCTAATCAGAGCAG AGATCCCCGCCCATGGGTGACCACCGAATCTGGTTGGTCAGGTCGTGTGGATGCGGA ATCCTTTACTAATCCGCCGATCACCATTGGGCATATGCGTCTGCCTCGCCAAGCTATT GAAGAGCCTCGCCGCAATGCCCGGCGCGTCCAGGAACTGTTTAGGCGTGAGCACTTC AAGCGACCCAACAAGATGCGGGAGTTTGCTCGGGTTCGTCCCAATGAGGCAGTGAC CAAACTGCGTAATGCTATTCGGGATCATGAGGCGTATCATTGGCCTGATCGGGAGCA CTTGGCTCGCACCGCGGAGCGTATGATCCGTAAAGAACGTGATCTGGCTAAGTTGAC CGGCAATGTGGATAAAGCCAGGGAAACCCTCGGTAGGACGTTTGAGCGCATTTTGTC GCTGCTCAGTGAAATGGACTATGTGGATTACTCTAATCCAGATAATCCAGTGATCACT GATGAAGGTGAGCGTTTGGCGAAAATCCACAGTGAGGCAGACCTGTTGGTTGCTCAG TGCCTCAAGCGTGGCATTTGGGACAACCTTGATCCCGCAGAGCTCGCCGGTGTGGTG AGTATGTGCACGTTTGAAAATCGTCGCGAAACCGGTGGTGAGGCTCAAGCTGTCACA GAGGCCATGGCTGATTCCATGAATAGCGTGGAACGTATTTGGGGTGAGCTGGTTGAA GATGAGCGCCGTCACCGTCTGCCAATTACTCGCCAGCCGGAAGCAGGTTTTGCCACC GCGATCCACCAGTGGGCTTCAGGTGCTCCGCTGGGATATTGCATGGCTGCGGCAGCG GAAAACGGCGCGGAGTTGACCCCTGGTGACTTCGTGCGCTGGTGCCGTCAAGTCATC GATCTTCTAGAGCAGGTTGCTAAGACTGCCTACTTTGATGAGACCACACGGAATGCTC GTCAGGCTATTGATGCGATTAGGCGTGGAGTTGTGGCGATCGGTTCCTAG |
| 35 | GTGACAACCAACGCCCCCGACGGAGCAACGAACAACATCAACAACGCACATTCGGG CGCTGTCGGGAAGCCAAAGGTACAGCTCAGCGATGCGGAAATTCAGAAATACACCG CAGCTTTCGCTGGCACCACCACAACCAAGCCATGGGAGCTGGAGGTGACCACAAAG TTTCTGAAAAAGATCGCGTGGGTAGCCGTGGTTGTCATCATGGCCGTTCACATCTTCA |
| | TGGGTGCCGTGGTGGACGTCGATTTCACCGGTGCAGCGGTCACCTTTGTTGACACTCT GGCATTCCCAGCGTTGGGCATCATCTTCTCCGTTCTTGTGTTCTTGGGACTGACTCGCC CTCGCGTGCGTGCCAACGAAGACGGCGTTGAGGTGCGTAACTTCATCGGAACTCGTT TCTACCCATGGGTTGTCATCTACGGCATGTCTTTCCCCAAGGGCAGCAGCGTGGCAC GTTTGGAGCTTCCAGACTTTGAATTCGTTCCCATGTGGGCTTTCCAGTCCCGCGATGG AGAAGATGTGGTGCGCGCGGTTGCGACCTTCCGCGACCTCGAAAACAAGTACATGCC AGAGGACTAA |
| 36 | ATGAAGCTTTTCTCCCGCACCTCACTGGTTGCACTTGGTACCGCTGCAGCTATCACTCT CTCCGGCGTAACCGCTCCTGCATTCGCTGATGAGGACTCCAACGCAGCTGTAAGTGC TCTTAAGACCGCTGAAGATAATACCCCTGAGGCGCCAGGGGCTTCTACTCCCCTGAA GCTTGAGCAGCCTGGCACCATCACCGGCGTCCCTGGCAAGGCGATCACCCCGGTCA CTGTTAAGGTCATCGCAGGCGAAGCAGAATCCTTCACCTCAGACAACCTTCCTTCTGG TCTCCTTATCGACAACACCGGAAAGATCACCGGTACTCCAAAGAAGGAATTCACTGG TTCCGCAAAGATCATTGCCAAGAACGAAGCAGGCGTTGAAGCAGAAGTTTACGTTAA CTTTGATTTCAACGAAGAGCCTTCCAGCGAAGAGCCTTCCAGCGGTTCTTCCGACACT GACAACATCGAAAACTGGATCAAGATCATCACAGCAGTCATCGGTGCACTAACCACC ATTTTAACCTTCAGTACCAAGCTGGATTCCTTCCTGAAGTAA |
| 37 | ATGGTCAGCCAAACGGAAAGACAGCATGCAATTGCTTCTTTACTGGCACCAACTGGT GCGGTGTCTGTAGGAGATTTAGCCGAGCATTTCCATGTGACAACCGAAACAGTGCGA CGTGATCTTCGGATCATGGAGTCACTGGGTTTGTTGCAACGAGTTCACGGTGGCGCC ATTAGCCCAGAGCCCATGGGTACGTCTCCCCCTCGGCTGAAACCTGCCTTGGTTAAA GGAATGCCACCGGAACCCCGTGTTTTAGAACTTGCAGAAACTGCAGTTTCCCTCATCA CACCTCTAGCACGCAGCATTTTCCTGGATTCAGGTTTAGCGTGCACGGCGATTGCCAC GGTGTTGGGGGATCCTCCAGAAGATGCCAGGTGGACTGTTGTTACAAGTTCCCCCGG |
| | CGCTGTGATTGCCTTGTCCGCGACAGATGCCACCTCCACGGTGGTGCTGCACGGGCA GGTTCACGGTAATTGTTCTTCAATCATTGGGTCCACGGCAGTAGACATGATTTCGCAG TTGCGCGCTGATATCGCCTTCGTGGAGGTTGATGCGATTCAATCCGATACAAGTCTGT GCACGTTTTTCCCGGAGACGATTCCCATCAAGCAAGCCATGATCAAAAACGCGGCTT TCACAGTTGCTGTTCTCAGCCCGAGATCTCCCCAAGATCAAGAACTTCAACTTTTGAA GCACCCTTTTTCCACCTTGGCTGATTTTGATGCCCTTGTTACCGATGACCACACGCTAG ATTTTCCAGTTTTGCCCGACCACAACTTTCAGGTGGTAACCCCATGA |
| 38 | GTGACGCAGCAATTGAACCACGCAAAGGTAAATCAACATCCCGGTCAAGCCACCCTC CCAGAAACCGCAGAGGGGCAGGTTCGCACCTACGAGGTAAAAACCTACGGCTGTCA GATGAATGTGCACGATTCTGAGCGCCTTTCGGGCCTGCTCGAGGAGGCTGGATACGT TGCTGCTCCGGAGGACACCACTCCGGATCTTGTCGTATTTAATACGTGCGCCGTGCGT GAAAACGCCGATATGCGCCTCTATGGCACTTTGGGCAACCTGCGCAGCGTGAAAGAA AAGAACCCAGGCATGCAAATCGCTGTCGGTGGTTGTTTGGCTCAAAAAGACAAAGAT ACCGTGGTGAAAAAAGCACCGTGGGTGGACGTGGTGTTTGGTACCCACAACATTGGT TCCTTGCCAACCTTGCTTCAGCGCGCGGAGCACAATGCCCAAGCGGAAGTCGAAATT GTCGATTCCCTCGAGCAGTTCCCGTCAGTACTTCCTGCAAAGCGCGAGTCTGCTTACG CTGGTTGGGTGTCCGTATCAGTCGGATGTAACAACACCTGTACTTTCTGCATCGTTCC GTCGCTGCGCGGTAAAGAGCAGGACCGTCGACCAGGAGACATCCTCGCAGAGGTAC AAGCACTGGTGGATCAGGGAGTTACCGAGGTAACTCTACTTGGCCAAAACGTAAATG CTTACGGCGTGAACTTTGTTGATCCTGAGCTAGAGCGCGATCGCAGTGCATTTTCCAA GCTGCTTCGTGCCTGTGGTGAGATCGAAGGCCTCGAGCGGGTTCGCTTCACCAGCCC TCACCCTGCAGAATTCACCTCTGATGTCATTGACGCCATGGCAGAGACCCCAAACAT CTGCCCGCAGCTGCACATGCCACTGCAGTCCGGATCTGACAAGGTGCTCAAAGAGAT GCGCCGTTCCTACCGATCCAAGAAGTTCCTCTCCATCTTGGATGAGGTCCGTGCGAA |
| | GATCCCTCACGCCTCTATCACCACCGATATTATTGTCGGATTCCCTGGCGAAACAGAG GAGGATTTCCAAGCAACCCTCGACGTTGTCAAGAAGGCACGCTTTACTTCTGCTTACA CCTTCCAATACAGCCCACGCCCTGGCACCCCTGCAGCGGAATATGAAAACCAGCTTC CAAAAGAAGTTGTGCAGGAACGCTACGAGCGCCTCATGGTCGTTCAGGAACAAGTCT GCGAAGAAGAAAACCAAAAGCTCATCGGCACCACCGTCGAATTGCTGGTCCAGGCT GGCGGAGGCCGCAAGAACGATGCCACCAAGCGCATGAGTGGTCGCGCACGCGATG GACGCCTCGTGCACTTTGCGCCAGAGGGCGACATTGATGGTGAGATCCGCCCCGGC GATTTTGTCACTGTCACGGTGACTGAGGCCAAGCCTTTCTTCCTCATCGCAGACTCCG GTGTGCAGACCCACCGCCGCACCAAAGCTGGTGACAACTCTGCAGTTGGTCAAGTTC CAACCACAGCACCGATCGGTGTGGGCTTGGGACTGCCACAAATCGGCGCACCAAAG GTGGCTCCTGCCACAGAATCTGCCTGCTGCTCCATTAACTAA |
| 39 | GTGGCTGTCGCAAAAGACGTGCTTAGTTTTTCACGACCCATTCCCGCACTTCACGCCA TCATCGGCGCGCTTGGATGGTTTGGAATGTTCGCAGTTAGTCTGTGGAGCACGCTGTA A |
| 40 | ATGAGCATCACCACACACGTCCAAGCACTCACCACAGCACTCAACGCCATCGACAAC CATTTGGCCAGCATGCTCGACCATGGTGTCACCCCAGACCAATACAAGGCCATCGAG CCCGACCTCATCGCCCTAGAACACACCATCAACCACCACGCCACCATCGCCGCCCAA ACCACAGCCCTCGCCGAACGCACCAACGCCGCCCACACCATTGGCTCCACCCACCTC ATCGACTACCTCACCACCACCTTCGGACTATCCAAAGCACGCGCCCACCACCGCATC AATCTCGCCCACTCCCTCTACCCCATACCGAAGCCAAACCCTGGATCTGGCAACGGC GGTAATGGTGGCAATCCCGACGGTGGTCCTGATGGTGGCGACTCGGGTGATGACGA CTCCGGCGACGATGACCCCGACCCCGAACCGGACAAGCCTGAAGACGGCAAACCTG ATGGTGATAAGCCTCGTGGGCCACGGATCAGCGCGGAAAAACACGCCATCATCACC GACGAACTCGCCCGCCTCAACCCGAATACCACACCCAGCGCCGAGGAGCTACGCAC |
| | CCAAGCCCTGAGTCAAGCGATCTGGCGCACCCCAGAAGACCTCCGCACGTGGCTAC GCCACCACGTCACCACCGCCAACAAAAACAACCCCAACCCCATCACCGCCATGAAA AGGCGCTACCTCGCTGTAGGTAAACCCGATGCCGACAACATGGTCCGCATCAGCGG CCTCGTGCCCGCAGCCACCGCAGCACTGATCACCGCGAACACCGCACCGTTAACCAA ACGCGGCAACCTCGTGGATCTACCCGCAGCAGAAGATATGCGCACCCGCGGGCAAC GCCATGCGGACGCGTTGCATCACATCATGGAGATCTACAACCACGGTATTGTCACCC CAGCTCGTGGTGGAACAGCCAGCATCATCATCTCCATGACCACCGATGATCTTGATG AGATCAACCACGGTGATCATGGCGATGGCAGTCTACTTAACAACCTGTACCCCACCA ATACGGGTTACTCATTGAACTTGGCGGAGATCATGAACCTCATCGCTGCGAAATACG ACTTCGCTGTGCTCCTCGATGGTGAGACGGGGCAGCCGTTGAACGTCAATAGGATGC AGCGCTCAGCGAACCTGACTCAACGCATCGCGTTGTTTGCTTCTGAGTTGGTGTGCTC GGCACCCAATTGTGACAGGCCGCAGTTAGAGTGCGAGGTTCATCATTTAGATCCCTG GATGAGAGGTGGGCTGACCAACCTGGTCAATCTCACGCATCAGTGCTTTAATCACCA CCCACGCAACGATGATTCCAGGAGTGGGGTCAATGGTAAAGGGTTTATGGACCGCG ATCCTGCTACTGGAAGAGTGGGGCACTACCCAGTGGGTGGTGAGGGGCCGGTGTTTA ACCGGTCGGCTGCTGCTGATCGTTCCGGTGGTGCGTGGGCGAGGCGTAAGCATCATG GCCCGCCACCCACACCGCCGGACCCGCCGGATCCGCCTGGTCCTGAGCCGGATCCT GGTGATGAGGGCAGATTGTTCTAG |
| 41 | GTGCGTATGTTGTTTGAGAACTCAATAGTGTGCCATTTATTATTTTTGTCACTACCACA GATCACCTTGGGTGATGTGTGGTGGGTCATGCCGGGTGGTGGATCGCCAAATTATCG ATCACTGTAA |
| 42 | ATGCCTAAACCCCTACCCCCAGAAACCCGACGCAAGATCATCGATTTCGATCCGTTC GCACCGAACAGCCCCTCGATCGAAGAGTTCTGCAGTCGGCTAAAAATATCGCGGCGC AGCTTCTACAACATCCGCAACCGATACCAACAAGACGCCAACGCAGCACTGCATCCA |
| | CACTCCAGCGCCCCAATCACCGCCCGGCGAACGTACGATGAATCCATCACCAGCACC TTGCTGTCCATCCGCGCACGCCTGAAAGCCCAAGGATGGGAGTACGGCCCGATCTCT ATTCGATTCGAAGGCATCTCCACCGGGGAACTGACTGCACCGATTCCATCTGTTTCAA CCATTGCTCGTTTGTTACGCGCCGCAGGAGCTGTTGAAAGTAACCCTAAGAAGCGAC CGAAGTCGTCGGTGGTGAGGTTTCAACGAGGTCAAGCCATGGAAATGTGGCAAATA GATGGTTTCATCTACACACTGCACGATACTGATCTGACTCGGGTAACGATCTATCAAA TCCTTGATGATGCCACTCGGTTTGATGTCGGAACCTGTGTCTTCCCCGCGAATGAAAA CTCAGTCGATGCCCGAACAGCGCTAGAACAAGCGATTGCTCATTTCGGTGCCCCGCA TGAATTACTCAGTGATAACGGCTCAGCGTTTAACCGGATGCGGCAAGGATACGTCGG AAGCTTGGAAAGCTATCTCGCGACGGTGGGGTGTTTAAGCATTACCGGCAAGCCTGG GCATCCTCAAACCCAAGGTAAAAACGAACGCAGCCACCGCACACTATTTCGTTTCCTT CAAGCTCATCAACCACACACACTTGAGGAATGTGCGCACTATATTGAACAGTTCCGC GATCATTACAACAATCGTCGCCCGCATCAGGGGTTGCCGAATAATCTCTCCCCGGCC GCGGCCTGGGAGATTGTGGGCTGTGTGGAACAACAACCCCCGATTGATCCCGTCGTG CTTCAACAGCAAGCCGATCACTATGCCCGTCGTCGAATTGAGAAGCAATCAGATGTC CCGCTTGTTCGGCAGGAGGAATCTGTTCCGGTGTCTAAGATGCAGCAGACCGAGCAA CGTCGACGGGTACGTCGTTTTGGTAAAAAGCCGGTGGATCCTGATGTGTTTACGTTTG CTGGGAATAATCAGAAGGTGGTGTTCCAGGGGATGCGTATTTCTGTTCCTCGTACGAT GAGGGACAGGGAGTTTTATCGGACGGTGACGGCAACTGAGCTTGGTTTCTGGGATGC GATAACCGGGGAGCTTGAGTTGTCGATTCCGTTGCCGGTTGTGGCGATTGCCCGGGG TAAGTCGTATATCAATTCTTACAACATTCGTGGGGTGTGGATGCAACACCCGACACCG TTGTGGCAACGCAAACGTGATGAGGCTGAGAAGCGATTCAAGTCGATAGATCCGGG GGATCTTCTGCGCTAG |
| 43 | ATGACTACTGCTGCAATCAGGGGCCTTCAGGGCGAGGCGCCGACCAAGAATAAGGA |
| | ACTGCTGAACTGGATCGCAGACGCCGTCGAGCTCTTCCAGCCTGAGGCTGTTGTGTT CGTTGATGGATCCCAGGCTGAGTGGGATCGCATGGCGGAGGATCTTGTTGAAGCCGG TACCCTCATCAAGCTCAACGAGGAAAAGCGTCCGAACAGCTACCTAGCTCGTTCCAA CCCATCTGACGTTGCGCGCGTTGAGTCCCGCACCTTCATCTGCTCCGAGAAGGAAGA AGATGCTGGCCCAACCAACAACTGGGCTCCACCACAGGCAATGAAGGACGAAATGT CCAAGCATTACGCTGGTTCCATGAAGGGGCGCACCATGTACGTCGTGCCTTTCTGCAT GGGTCCAATCAGCGATCCGGACCCTAAGCTTGGTGTGCAGCTCACTGACTCCGAGTA CGTTGTCATGTCCATGCGCATCATGACCCGCATGGGTATTGAAGCGCTGGACAAGAT CGGCGCGAACGGCAGCTTCGTCAGGTGCCTCCACTCCGTTGGTGCTCCTTTGGAGCC AGGCCAGGAAGACGTTGCATGGCCTTGCAACGACACCAAGTACATCACCCAGTTCCC AGAGACCAAGGAAATTTGGTCCTACGGTTCCGGCTACGGCGGAAACGCAATCCTGG CAAAGAAGTGCTACGCACTGCGTATCGCATCTGTCATGGCTCGCGAAGAAGGATGGA TGGCTGAGCACATGCTCATCCTGAAGCTGATCAACCCAGAGGGCAAGGCGTACCAC ATCGCAGCAGCATTCCCATCTGCTTGTGGCAAGACCAACCTCGCCATGATCACTCCAA CCATCCCAGGCTGGACCGCTCAGGTTGTTGGCGACGACATCGCTTGGCTGAAGCTGC GCGAGGACGGCCTCTACGCAGTTAACCCAGAAAATGGTTTCTTCGGTGTTGCTCCAG GCACCAACTACGCATCCAACCCAATCGCGATGAAGACCATGGAACCAGGCAACACC CTGTTCACCAACGTGGCACTCACCGACGACGGCGACATCTGGTGGGAAGGCATGGA CGGCGACGCCCCAGCTCACCTCATTGACTGGATGGGCAACGACTGGACCTCAGAGTC CGACGAAAACGCTGCTCACCCTAACTCCCGTTACTGCGTAGCAATCGACCAGTCCCC AGCAGCAGCACCTGAGTTCAACGACTGGGAAGGCGTCAAGATCGACGCAATCCTCTT CGGTGGACGTCGCGCAGACACCGTCCCACTGGTTACCCAGACCTACGACTGGGAGC ACGGCACCATGGTTGGTGCACTGCTCGCATCCGGTCAGACCGCAGCTTCCGCAGAAG CAAAGGTCGGCACACTCCGCCACGACCCAATGGCAATGCTCCCATTCATTGGCTACA |
| | ACGCTGGTGAATACCTGCAGAACTGGATTGACATGGGTAACAAGGGTGGCGACAAG ATGCCATCCATCTTCCTGGTCAACTGGTTCCGCCGTGGCGAAGATGGACGCTTCCTGT GGCCTGGCTTCGGCGACAACTCTCGCGTTCTGAAGTGGGTCATCGACCGCATCGAAG GCCACGTTGGCGCAGACGAGACCGTTGTTGGACACACCGCTAAGGCCGAAGACCTC GACCTCGACGGCCTCGACACCCCAATTGAGGATGTCAAGGAAGCACTGACCGCTCCT GCAGAGCAGTGGGCAAACGACGTTGAAGACAACGCCGAGTACCTCACTTTCCTCGG ACCACGTGTTCCTGCAGAGGTTCACAGCCAGTTCGATGCTCTGAAGGCCCGCATTTCA GCAGCTCACGCTTAA |
| 44 | ATGACAACCGCAGTAGATCAAAACTCACCGCCCAAGCAGCAACTCAACAAGCGCGT CCTGCTGGGCAGCTTGAGTGGCAGCGTTATCGAATGGTTCGACTTCCTGGTTTACGGA ACCGTCGCCGCGCTGGTCTTCAACAAGATGTACTTCCCCAGCGGCAACGAGTTCCTCT CCACAATCCTGGCGTACGCATCCTTCTCCCTGACCTTCTTCTTCCGCCCCATTGGTGGC GTCATCTTCGCCCACATCGGCGACCGCATTGGGCGTAAGAAGACCCTGTTCATCACC TTGATGCTCATGGGTGGCGGCACCGTCGCCATTGGTTTGCTGCCCGACTACAACGCC ATCGGCATTTGGGCACCAATCCTTCTGATGTTCCTCCGCATTTTGCAGGGCATCGGAA TTGGCGGCGAATGGGGTGGCGCACTGCTCCTGGCATACGAATACGCTCCAAAGAAG CAGCGTGGGCTCTACGGCGCAGTTCCTCAAATGGGCATTTCCCTGGGCATGCTGCTT GCAGCTGGCGTGATCTCTCTGCTCACCCTCATGCCGGAAGATCAGTTCCTCACCTGGG GCTGGCGCATCCCATTCGTCGGATCCATCCTCCTAGTGTTCATCGGCCTGTTCATCCG AAACGGCCTTGATGAAACCCCCGAGTGCAAGCGTATCCGCGATTCCGGCCAGCAGG TAAAGATGCCTCTGAAGGAAGTTCTGACCAAGTACTGGCCAGCCGTTCTGGTCTCCAT CGGCGCAAAAGCTGCCGAGACCGGCCCCTTCTACATCTTCGGCACCTACATCGTTGC TTACGCAACCAACTTCCTGAACATCCGCGACAACATTGTCCTTCTGGCAGTTGCTTGC GCCGCCCTCGTTGCCACCATCTGGATGCCACTGTTCGGATCCTTCTCCGACCGCGTCA |
| | ACCGTGCAGTGCTCTACAGGATCTGTGCATCCGCAACCATCGTGCTGATTGTCCCTTA CTACTTGGTCCTCAACACCGGCGAAATTTGGGCACTGTTTATCACTACCGTGATTGGC TTCGGCATCCTCTGGGGTAGCGTCAACGCAATCCTCGGAACCGTCATCGCAGAAAAC TTCGCACCTGAGGTCCGCTACACCGGCGCTACCCTGGGTTACCAAGTCGGAGCAGCA CTCTTCGGCGGTACCGCACCCATTATCGCAGCATGGCTGTTCGAAATCTCCGGCGGA CAATGGTGGCCAATCGCCGTCTACGTCGCTGCATGTTGCCTTCTCTCTGTGATCGCCT CGTTCTTCATCCAACGCGTCGCGCACCAAGAGAACTAA |

### Example 3: Construction of mutant genes-introduced L-lysine producing strains

### Example 3-1: Construction of recombinant vectors for introducing mutant genes

In order to confirm the effects of some variants identified in Example 2, vectors capable of introducing them on chromosome were constructed.

Specifically, to insert each of NCgl0030, NCgl0288, NCgl0337, NCgl0458, NCgl0686, NCgl0706, NCgl0799, NCgl1165, NCgl1262, NCgl1432, NCgl1531, NCgl1588, NCgl1859, NCgl1874 and NCgl2356 gene mutations into CJ3P, vectors comprising target mutations were constructed.

Specifically, the genomic DNA of the CJ3P_mt5 strain was extracted according to the protocol provided in the kit using G-spin Total DNA extraction mini kit (Intron company, Cat. No 17045), and PCR was conducted using the genomic DNA as a template. As polymerase, Solg^{™} Pfu-X DNA polymerase was used, and the PCR conditions were denaturation at 95°C for 4 minutes; and then repeating denaturation at 95°C for 30 seconds, annealing at 60°C for 30 seconds, and polymerization at 72°C for 50 seconds 27 times; and then performing polymerization at 72°C for 5 minutes. The primer sequences used in the above experiment were shown in Table 4 below.

**[Table 4]**

| primer | sequence (5'->3') | SE Q ID N O: |
|---|---|---|
| NCgl0030*_F | tgaattcgagctcggtacccCCTGCTGATGATCGCTGATG | 45 |
| NCgl0030*_R | tcgactctagaggatccccCATTCCAAAGATGAGTGCAC | 46 |
| NCgl0288*_F | tgaattcgagctcggtacccCAAAAAGCATCAGCAAGAGC | 47 |
| NCgl0288*_R | tcgactctagaggatccccATGTGAAAACATGGTCCACT | 48 |
| NCgl0337*_F | tgaattcgagctcggtacccTTTCGTGCGTTACGTACTAA | 49 |
| NCgl0337*_R | tcgactctagaggatccccGCTATTGCTCATTGTTGGCC | 50 |
| NCgl0458*_F | tgaattcgagctcggtacccCACTTGGTGACACCGATGAG | 51 |
| NCgl0458*_R | tcgactctagaggatccccTAAGTGGTGCAGTTTAGCTA | 52 |
| NCgl0686*_F | tgaattcgagctcggtacccCCACGCATTATTTCCGGACA | 53 |
| NCgl0686*_R | tcgactctagaggatccccCCAGACCACTGGAACGCTGA | 54 |
| NCgl0706*_F | tgaattcgagctcggtacccCGTGGCTACTTCTGCGATCC | 55 |
| NCgl0706*_R | tcgactctagaggatccccGTGCGGCCCCTGCAAAATGG | 56 |
| NCgl0799*_F | tgaattcgagctcggtacccCGCCAGAAGATGGAAGGTTA | 57 |
| NCgl0799*_R | tcgactctagaggatccccGGCCACCAGACACCTTGACG | 58 |
| NCgl1165*_F | tgaattcgagctcggtacccATTGCTTCCAAGGGTATTTA | 59 |
| NCgl1165*_R | tcgactctagaggatccccTCAACCTCATTCGCCCAGAC | 60 |
| NCgl1262*_F | tgaattcgagctcggtacccACCCCACTGCGCGACATCAA | 61 |
| NCgl1262*_R | tcgactctagaggatccccGACAGCGCGGAAGCCGTAGT | 62 |
| NCgl1432*_F | tgaattcgagctcggtacccATGCCTGGTAGAAAACGACC | 63 |
| NCgl1432*_R | tcgactctagaggatccccTCAGCCATGGCCTCTGTGAC | 64 |
| NCgl1531*_F | tgaattcgagctcggtacccGCTCTTGATACTTCCACCCC | 65 |
| NCgl1531*_R | tcgactctagaggatccccTTGGGGAAAGACATGCCGTA | 66 |
| NCgl1588*_F | tgaattcgagctcggtacccTTAAATAACATCTTGCTTGA | 67 |
| NCgl1588*_R | tcgactctagaggatccccGATTCACAAAGGATTAGTCG | 68 |
| NCgl1859*_F | tgaattcgagctcggtacccCACTTTTAATAACTTCATAC | 69 |
| NCgl1859*_R | tcgactctagaggatccccCAGACTTGTATCGGATTGAA | 70 |
| NCgl1874*_F | tgaattcgagctcggtacccGCGCCTTTCGGGCCTGCTCG | 71 |
| NCgl1874*_R | tcgactctagaggatccccGTCAATGACATCAGAGGTGA | 72 |
| NCgl2356*_F | tgaattcgagctcggtacccGTTGCATCACATCATGGAGA | 73 |
| NCgl2356*_R | tcgactctagaggatccccTACCGAACGGGCCGGAGCCG | 74 |

The obtained mutation-introduced fragments and pDC24 vector (SEQ ID NO: 107, Table 5) treated with restriction enzyme smal were cloned using Gibson assembly method (DG Gibson et al., NATURE METHODS, VOL.6 NO.5, MAY 2009, NEBuilder HiFi DNA Assembly Master Mix) to obtain recombinant plasmids, and named as vectors PDC24-NCgl0030*, PDC24-NCgl0288*, PDC24-NCgl0337*, PDC24-NCgl0458*, PDC24-NCgl0686*, PDC24-NCgl0706*, PDC24-NCgl0799*, PDC24-NCgl1165*, PDC24-NCgl1262*, PDC24-NCgl1432*, PDC24-NCgl1531*, PDC24-NCgl1588*, PDC24-NCgl1859*, PDC24-NCgl1874* and PDC24-NCgl2356* comprising each mutation-introduced fragment.

**[Table 5]**

| |
|---|
| Sequence of pDC24 (SEQ ID NO: 107) (5'→3') |
| GTTCGCTTGCTGTCCATAAAACCGCCCAGTCTAGCTATCGCCATGTAAGCCCACTGCAAGCT ACCTGCTTTCTCTTTGCGCTTGCGTTTTCCCTTGTCCAGATAGCCCAGTAGCTGACATTCATC CGGGGTCAGCACCGTTTCTGCGGACTGGCTTTCTACGTGTTCCGCTTCCTTTAGCAGCCCTT |
| GCGCCCTGAGTGCTTGCGGCAGCGTGAAGCTAGCTTTTATCGCCATTCGCCATTCAGGCTG CGCAACTGTTGGGAAGGGCGATCGGTGCGGGCCTCTTCGCTATTACGCCAGCTGGCGAAA GGGGGATGTGCTGCAAGGCGATTAAGTTGGGTAACGCCAGGGTTTTCCCAGTCACGACGTT GTAAAACGACGGCCAGTGAATTCGAGCTCGGTACCCGGGGATCCTCTAGAGTCGACCTGC AGGCATGCAAGCTTGGCGTAATCATGGTCATAGCTGTTTCCTGTGTGAAATTGTTATCCGCT CACAATTCCACACAACATACGAGCCGGAAGCATAAAGTGTAAAGCCTGGGGTGCCTAATG AGTGAGCTAACTCACATTAATTGCGTTGCGCTCACTGCCCGCTTTCCAGTCGGGAAACCTGT CGTGCCAGCTGCATTAATGAATCGGCCAACGCGCGGGGAGAGGCGGTTTGCGTATTGGGC GCTCTTCCGCTTCCTCGCTCACTGACTCGCTGCGCTCGGTCGTTCGGCTGCGGCGAGCGGT ATCAGCTCACTCAAAGGCGGTAATACGGTTATCCACAGAATCAGGGGATAACGCAGGAAA GAACATGTGAGCAAAAGGCCAGCAAAAGGCCAGGAACCGTAAAAAGGCCGCGTTGCTGG CGTTTTTCCATAGGCTCCGCCCCCCTGACGAGCATCACAAAAATCGACGCTCAAGTCAGAG GTGGCGAAACCCGACAGGACTATAAAGATACCAGGCGTTTCCCCCTGGAAGCTCCCTCGT GCGCTCTCCTGTTCCGACCCTGCCGCTTACCGGATACCTGTCCGCCTTTCTCCCTTCGGGAA GCGTGGCGCTTTCTCAATGCTCACGCTGTAGGTATCTCAGTTCGGTGTAGGTCGTTCGCTCC AAGCTGGGCTGTGTGCACGAACCCCCCGTTCAGCCCGACCGCTGCGCCTTATCCGGTAACT ATCGTCTTGAGTCCAACCCGGTAAGACACGACTTATCGCCACTGGCAGCAGCCACTGGTAA CAGGATTAGCAGAGCGAGGTATGTAGGCGGTGCTACAGAGTTCTTGAAGTGGTGGCCTAA CTACGGCTACACTAGAAGGACAGTATTTGGTATCTGCGCTCTGCTGAAGCCAGTTACCTTCG GAAAAAGAGTTGGTAGCTCTTGATCCGGCAAACAAACCACCGCTGGTAGCGGTGGTTTTT TTGTTTGCAAGCAGCAGATTACGCGCAGAAAAAAAGGATCTCAAGAAGATCCTTTGATCTTT TCTACGGGGTCTGACGCTCAGTGGAACGAAAACTCACGTTAAGGGATTTTGGTCATGAGAT TATCAAAAAGGATCTTCACCTAGATCCTTTTGGGGTGGGCGAAGAACTCCAGCATGAGATC CCCGCGCTGGAGGATCATCCAGCCCTGATAGAAACAGAAGCCACTGGAGCACCTCAAAAA |
| CACCATCATACACTAAATCAGTAAGTTGGCAGCATCACCCGACGCACTTTGCGCCGAATAA ATACCTGTGACGGAAGATCACTTCGCAGAATAAATAAATCCTGGTGTCCCTGTTGATACCG GGAAGCCCTGGGCCAACTTTTGGCGAAAATGAGACGTTGATCGGCACGTAAGAGGTTCCA ACTTTCACCATAATGAAATAAGATCACTACCGGGCGTATTTTTTGAGTTATCGAGATTTTCAG GAGCTGATAGAAACAGAAGCCACTGGAGCACCTCAAAAACACCATCATACACTAAATCAG TAAGTTGGCAGCATCACCCGACGCACTTTGCGCCGAATAAATACCTGTGACGGAAGATCAC TTCGCAGAATAAATAAATCCTGGTGTCCCTGTTGATACCGGGAAGCCCTGGGCCAACTTTT GGCGAAAATGAGACGTTGATCGGCACGTAAGAGGTTCCAACTTTCACCATAATGAAATAAG ATCACTACCGGGCGTATTTTTTGAGTTATCGAGATTTTCAGGAGCTCTTTGGCATCGTCTCTC GCCTGTCCCCTCAGTTCAGTAATTTCCTGCATTTGCCTGTTTCCAGTCGGTAGATATTCCACA AAACAGCAGGGAAGCAGCGCTTTTCCGCTGCATAACCCTGCTTCGGGGTCATTATAGCGAT TTTTTCGGTATATCCATCCTTTTTCGCACGATATACAGGATTTTGCCAAAGGGTTCGTGTAGA CTTTCCTTGGTGTATCCAACGGCGTCAGCCGGGCAGGATAGGTGAAGTAGGCCCACCCGC GAGCGGGTGTTCCTTCTTCACTGTCCCTTATTCGCACCTGGCGGTGCTCAACGGGAATCCTG CTCTGCGAGGCTGGCCGGCTACCGCCGGCGTAACAGATGAGGGCAAGCGGATGGCTGAT GAAACCAAGCCAACCAGGAAGGGCAGCCCACCTATCAAGGTGTACTGCCTTCCAGACGAA CGAAGAGCGATTGAGGAAAAGGCGGCGGCCGGCATGAGCCTGTCGGCCTACCTGCT GGCCGTCGGCCAGGGCTACAAAATCACGGGCGTCGTGGACTATGAGCACGTCCGCGAGG GCGTCCCGGAAAACGATTCCGAAGCCCAACCTTTCATAGAAGGCGGCGGTGGAATCGAAA TCTCGTGATGGCAGGTTGGGCGTCGCTTGGTCGGTCATTTCGAAAAAGGTTAGGAATACGG TTAGCCATTTGCCTGCTTTTATATAGTTCATATGGGATTCACCTTTATGTTGATAAGAAATAA AAGAAAATGCCAATAGGATATCGGCATTTTCTTTTGCGTTTTTATTTGTTAACTGTTAATTGTC CTTGTTCAAGGATGCTGTCTTTGACAACAGATGTTTTCTTGCCTTTGATGTTCAGCAGGAAGC TCGGCGCAAACGTTGATTGTTTGTCTGCGTAGAATCCTCTGTTTGTCATATAGCTTGTAATCA |
| CGACATTGTTTCCTTTCGCTTGAGGTACAGCGAAGTGTGAGTAAGTAAAGGTTACATCGTTA GGCGGATCAAGATCCATTTTTAACACAAGGCCAGTTTTGTTCAGCGGCTTGTATGGGCCAG TTAAAGAATTAGAAACATAACCAAGCATGTAAATATCGTTAGACGTAATGCCGTCAATCGT CATTTTTGATCCGCGGGAGTCAGTGAACAGGTACCATTTGCCGTTCATTTTAAAGACGTTCG CGCGTTCAATTTCATCTGTTACTGTGTTAGATGCAATCAGCGGTTTCATCACTTTTTTCAGTGT GTAATCATCGTTTAGCTCAATCATACCGAGAGCGCCGTTTGCTAACTCAGCCGTGCGTTTTT TATCGCTTTGCAGAAGTTTTTGACTTTCTTGACGGAAGAATGATGTGCTTTTGCCATAGTATG CTTTGTTAAATAAAGATTCTTCGCCTTGGTAGCCATCTTCAGTTCCAGTGTTTGCTTCAAATA CTAAGTATTTGTGGCCTTTATCTTCTACGTAGTGAGGATCTCTCAGCGTATGGTTGTCGCCTG AGCTGTAGTTGCCTTCATCGATGAACTGCTGTACATTTTGATACGTTTTTCCGTCACCGTCAA AGATTGATTTATAATCCTCTACACCGTTGATGTTCAAAGAGCTGTCTGATGCTGATACGTTA ACTTGTGCAGTTGTCAGTGTTTGTTTGCCGTAATGTTTACCGGAGAAATCAGTGTAGAATAA ACGGATTTTTCCGTCAGATGTAAATGTGGCTGAACCTGACCATTCTTGTGTTTGGTCTTTTAG GATAGAATCATTTGCATCGAATTTGTCGCTGTCTTTAAAGACGCGGCCAGCGTTTTTCCAGC TGTCAATAGAAGTTTCGCCGACTTTTTGATAGAACATGTAAATCGATGTGTCATCCGCATTTT TAGGATCTCCGGCTAATGCAAAGACGATGTGGTAGCCGTGATAGTTTGCGACAGTGCCGTC AGCGTTTTGTAATGGCCAGCTGTCCCAAACGTCCAGGCCTTTTGCAGAAGAGATATTTTTAA TTGTGGACGAATCAAATTCAGAAACTTGATATTTTTCATTTTTTTGCTGTTCAGGGATTTGCA GCATATCATGGCGTGTAATATGGGAAATGCCGTATGTTTCCTTATATGGCTTTTGGTTCGTTT CTTTCGCAAACGCTTGAGTTGCGCCTCCTGCCAGCAGTGCGGTAGTAAAGGTTAATACTGTT GCTTGTTTTGCAAACTTTTTGATGTTCATCGTTCATGTCTCCTTTTTTATGTACTGTGTTAGCG GTCTGCTTCTTCCAGCCCTCCTGTTTGAAGATGGCAAGTTAGTTACGCACAATAAAAAAAGA CCTAAAATATGTAAGGGGTGACGCCAAAGTATACACTTTGCCCTTTACACATTTTAGGTCTT GCCTGCTTTATCAGTAACAAACCCGCGCGATTTACTTTTCGACCTCATTCTATTAGACTCTCG |
| TTTGGATTGCAACTGGTCTATTTTCCTCTTTTGTTTGATAGAAAATCATAAAAGGATTTGCAG ACTACGGGCCTAAAGAACTAAAAAATCTATCTGTTTCTTTTCATTCTCTGTATTTTTTATAGTT TCTGTTGCATGGGCATAAAGTTGCCTTTTTAATCACAATTCAGAAAATATCATAATATCTCAT TTCACTAAATAATAGTGAACGGCAGGTATATGTGATGGGTTAAAAAGGATCACCCCAGAGT CCCGCTCAGAAGAACTCGTCAAGAAGGCGATAGAAGGCGATGCGCTGCGAATCGGGAGC GGCGATACCGTAAAGCACGAGGAAGCGGTCAGCCCATTCGCCGCCAAGCTCTTCAGCAAT ATCACGGGTAGCCAACGCTATGTCCTGATAGCGGTCCGCCACACCCAGCCGGCCACAGTC GATGAATCCAGAAAAGCGGCCATTTTCCACCATGATATTCGGCAAGCAGGCATCGCCATGG GTCACGACGAGATCCTCGCCGTCGGGCATCCGCGCCTTGAGCCTGGCGAACAGTTCGGCT GGCGCGAGCCCCTGATGCTCTTCGTCCAGATCATCCTGATCGACAAGACCGGCTTCCATCC GAGTACGTGCTCGCTCGATGCGATGTTTCGCTTGGTGGTCGAATGGGCAGGTAGCCGGATC AAGCGTATGCAGCCGCCGCATTGCATCAGCCATGATGGATACTTTCTCGGCAGGAGCAAG GTGAGATGACAGGAGATCCTGCCCCGGCACTTCGCCCAATAGCAGCCAGTCCCTTCCCGCT TCAGTGACAACGTCGAGACAGCTGCGCAAGGAACGCCCGTCGTGGCCAGCCACGATAGCC GCGCTGCCTCGTCTTGGAGTTCATTCAGGGCACCGGACAGGTCGGTCTTGACAAAAAGAAC CGGGCGCCCCTGCGCTGACAGCCGGAACACGGCGGCATCAGAGCAGCCGATTGTCTGTTG TGCCCAGTCATAGCCGAATAGCCTCTCCACCCAAGCGGCCGGAGAACCTGCGTGCAATCC ATCTTGTTCAATCATGCGAAACGATCCTCATCCTGTCTCTTGATCAGATCTTGATCCCCTGCG CCATCAGATCCTTGGCGGCAAGAAAGCCATCCAGTTTACTTTGCAGGGCTTCCCAACCTTA CCAGAGGGCGCCCCAGCTGGCAATTCCG |

### Example 3-2: Construction of mutant gene-introduced L-lysine production strains

The 15 kinds of vectors constructed in Example 3-1 were transformed into a lysine production strain, CJ3P, and strains in which the vectors were inserted on chromosome by recombination of homologous sequences were selected through a kanamycin medium. After that, for the transformed strains in which secondary recombination was completed, mutant gene-introduced strains were confirmed by PCR using the primer pairs of Table 6 below.

**[Table 6]**

| primer | sequence (5'→3') | SEQ ID NO: |
|---|---|---|
| NCgl0030_F | GGCGCCGTGCTTGTTTTTAC | 75 |
| NCgl0030_R | ATCGCTCCTTGTTGAAACGC | 76 |
| NCgl0288_F | CCTGCACGTCACCGTCATTT | 77 |
| NCgl0288_R | GCCTCAAATGGATCTGACGG | 78 |
| NCgl0337_F | TTGCGCGATATTGAAGAGGT | 79 |
| NCgl0337_R | GCCCCCGGAAGCTCAAGTTC | 80 |
| NCgl0458_F | CTGGAAGAAGAGACCCTAGA | 81 |
| NCgl0458_R | CTGATCTTTACGTAACCGGG | 82 |
| NCgl0686_F | CCCCAGAAGCACCCGTTGTG | 83 |
| NCgl0686_R | CGCCATTGGCCACATCCAAG | 84 |
| NCgl0706_F | GATCTGCTTGCTGAACATGAT | 85 |
| NCgl0706_R | TCATAAGTGGGCTTTGTTTG | 86 |
| NCgl0799_F | GTCTACGTTCTGGTTGCATC | 87 |
| NCgl0799_R | AGCTGAGTGGTCAGAGTTGC | 88 |
| NCgl1165_F | TTACCAGCCAACCCTGGCTG | 89 |
| NCgl1165_R | ACTACTTCAGCTAAACGGAG | 90 |
| NCgl1262_F | AATCGATGGCTCCGCACTGA | 91 |
| NCgl1262_R | ACAGAAGTTCGATGTCGGAC | 92 |
| NCgl1432_F | GCGTGAAGATCAAGACGCAG | 93 |
| NCgl1432_R | CTGGTGGATCGCGGTGGCAA | 94 |
| NCgl1531_F | GTAGTGCAAGAACTGGCACG | 95 |
| NCgl1531_R | CGCACCACATCTTCTCCATC | 96 |
| NCgl1588_F | GCAGGCAATAGGTAGTAAAT | 97 |
| NCgl1588_R | CTTAGGTTTAAATATTCGAA | 98 |
| NCgl1859_F | AAGCGATCACCATATTTCAT | 99 |
| NCgl1859_R | GGGCATCAAAATCAGCCAAG | 100 |
| NCgl1874_F | ATAGGCGTTTATGTCAGCTC | 101 |
| NCgl1874_R | GGGATCTTCGCACGGACCTC | 102 |
| NCgl2356_F | CAACCCCATCACCGCCATGA | 103 |
| NCgl2356_R | CACCGGTGAAAAGGAGAGAA | 104 |

| | | |
|---|---|---|
| The recombinant strains were named CJ3P△NCgl0030::NCgl0030*, CJ3P△NCgl0288::NCgl0288*, CJ3P△NCgl0337::NCgl0337*, CJ3P△NCgl0458::NCgl0458*, CJ3P△NCgl0686::NCgl0686*, CJ3P△NCgl0706::NCgl0706*, CJ3P△NCgl0799::NCgl0799*, CJ3P△NCgl1165::NCgl1165*, CJ3P△NCgl1262::NCgl1262*, CJ3P△NCgl1432::NCgl1432*, CJ3P△NCgl1531::NCgl1531*, CJ3P△NCgl1588::NCgl1588*, CJ3P△NCgl1859::NCgl1859*, CJ3P△NCgl1874::NCgl1874* and CJ3P△NCgl2356::NCgl2356*, respectively. | | |

### Example 4. Evaluation of lysine productivity of mutant gene-introduced L-lysine producing strains

In order to confirm the L-lysine productivity of the strains constructed in Example 3-2, it was evaluated by culturing them by the following method.

Each strain was inoculated into a 250 ml corner-baffled flask containing 25 ml of a seed medium, and it was cultured with shaking at 200 rpm at 30°C for 20 hours. Into a 250 ml corner-baffled flask containing 24 ml of a production medium, 1 ml of the seed cultured solution was inoculated, and it was cultured with shaking at 200 rpm at 30°C for 72 hours.

### <Seed medium (pH 7.0)>

Glucose 20 g, peptone 10 g, yeast extract 5 g, urea 1.5 g, KH₂PO₄ 4 g, K₂HPO₄ 8g, MgSO₄·7H₂O 0.5 g, biotin 0.1 mg, thiamine HCl 1 mg, calcium-pantothenate 2 mg, nicotinamide 2 mg (on the basis of 1 liter of distilled water)

### <Production medium (pH 7.0)>

Glucose 100 g, (NH₄)₂SO₄ 40 g, soybean protein 2.5 g, corn steep solids 5 g, urea 3 g, KH₂PO₄ 1 g, MgSO₄·7H₂O 0.5 g, biotin 100 ug, thiamine hydrochloride 1000 ug, calcium-pantothenate 2000 ug, nicotinamide 3000 ug, CaCO₃ 30 g (on the basis of 1 liter of distilled water)

After completing the culturing, the amount of produced L-lysine was measured using HPLC, and the analysis results were shown in Table 7 below.

**[Table 7]**

| strain | L-lysine (g/L) |
|---|---|
| CJ3P | 7.2 |
| CJ3P△NCgl0030::NCgl0030* | 7.7 |
| CJ3P△NCgl0288::NCgl0288* | 7.4 |
| CJ3P△NCgl0337::NCgl0337* | 7.4 |
| CJ3P△NCgl0458::NCgl0458* | 7.6 |
| CJ3P△NCgl0686::NCgl0686* | 7.9 |
| CJ3P△NCgl0706::NCgl0706* | 7.4 |
| CJ3P△NCgl0799::NCgl0799* | 7.9 |
| CJ3P△NCgl1165::NCgl1165* | 7.5 |
| CJ3P△NCgl1262::NCgl1262* | 7.4 |
| CJ3P△NCgl1432::NCgl1432* | 7.4 |
| CJ3P△NCgl1531::NCgl1531* | 7.6 |
| CJ3P△NCgl1588::NCgl1588* | 7.7 |
| **CJ3P△NCgl1859::NCgl1859*** | 8.3 |
| CJ3P△NCgl1874::NCgl1874* | 7.6 |
| CJ3P△NCgl2356::NCgl2356* | 7.5 |

As a result, as shown in Table 7 above, it was confirmed that most of the lysine production strains in which the gene variants were introduced had lysine productivity equal or more to the parent strain. In particular, it was confirmed that CJ3P△NCgl1859::NCgl1859* had the significantly increased lysine productivity compared to the parent strain CJ3P. Through this, it could be confirmed that L-lysine could be much more efficiently produced by introducing the variant in which the 75^{th} amino acid of the NCg11859 gene was substituted from glycine to valine.

From the above description, those skilled in the art to which the present disclosure pertains will be able to understand that the present invention can be implemented in other specific forms without changing the technical spirit or essential features thereof. In this regard, the examples described above should be understood as illustrative and non-limiting in all respects. The scope of the present disclosure should be construed that all changed or modified forms derived from the meaning and scope of the claims to be described later rather than the above detailed description and equivalent concepts thereof are included in the scope of the present disclosure.

## Claims

1. A protein variant comprising the amino acid sequence in which the amino acid corresponding to the 75th position from the N-terminus in the amino acid sequence of SEQ ID NO: 105 is substituted with valine.

2. The protein variant according to claim 1, wherein the protein variant has amino acid sequence identity of 95% or more to less than 100% to the amino acid sequence of SEQ ID NO: 105.

3. The protein variant according to claim 1, wherein the protein variant comprises the amino acid sequence of SEQ ID NO: 15.

4. A polynucleotide encoding the protein variant of Claim 1.

5. A microorganism of the genus of *Corynebacterium* comprising at least one selected from the group consisting of the protein variant according to Claim 1 and the polynucleotide encoding the protein variant.

6. The microorganism according to claim 5, wherein the microorganism of the genus of *Corynebacterium* is *Corynebacterium glutamicum.*

7. The microorganism according to claim 5, wherein the microorganism has increased L-lysine productivity, compared to a microorganism of the genus of *Corynebacterium* which does not comprise any one selected from the group consisting of the protein variant and the polynucleotide encoding the protein variant.

8. A composition for producing L-lysine, comprising a microorganism of the genus of *Corynebacterium* comprising at least one selected from the group consisting of a protein variant comprising the amino acid sequence in which the amino acid corresponding to the 75th position from the N-terminus in the amino acid sequence of SEQ ID NO: 105 is substituted with valine and the polynucleotide encoding the protein variant.

9. The composition for producing L-lysine according to claim 8, wherein the protein variant comprises the amino acid sequence of SEQ ID NO: 15.

10. The composition for producing L-lysine according to claim 8, wherein the microorganism of the genus of *Corynebacterium* is *Corynebacterium glutamicum.*

11. A method of producing L-lysine, comprising culturing the microorganism according to claim 5 in a medium.

12. The method of producing L-lysine according to claim 11, wherein the microorganism of the genus of *Corynebacterium* is *Corynebacterium glutamicum.*

13. The method of producing L-lysine according to claim 11, wherein the method further comprises recovering L-lysine from the cultured medium or microorganism.

14. The method of producing L-lysine according to claim 11, wherein the method is performed at pH 6 to pH 9.

15. A use for producing L-lysine, of the microorganism according to any one claim of claim 5 to claim 7.

16. A composition, method, product, process, or use **characterized by** at least one element disclosed in the present disclosure.
